(19) **Europäisches Patentamt**
**European Patent Office**
**Office européen des brevets**

(11) **EP 2 552 452 B1**

(12) **EUROPEAN PATENT SPECIFICATION**

(45) Date of publication and mention
of the grant of the patent:
**19.08.2015 Bulletin 2015/34**

(51) Int Cl.:
*A61K 31/675* *(2006.01)*  *A61K 45/06* *(2006.01)*
*A61K 31/517* *(2006.01)*  *A61K 31/522* *(2006.01)*
*A61K 31/662* *(2006.01)*  *A61K 39/395* *(2006.01)*
*A61P 35/00* *(2006.01)*

(21) Application number: **11709746.9**

(22) Date of filing: **24.03.2011**

(86) International application number:
**PCT/EP2011/054548**

(87) International publication number:
**WO 2011/120870 (06.10.2011 Gazette 2011/40)**

(54) **EGFR inhibitor and antiviral agent for simultaneous, separate or sequential use in the treatment and/or prevention and/or palliation of cancer**

EGFR-Inhibitor und antivirales Mittel zur gleichzeitigen, getrennten oder aufeinanderfolgenden Verwendung bei der Behandlung und/oder Vorbeugung und/oder Abschwächung von Krebs

Inhibiteur EGFR et agent antiviral pour une utilisation simultanée, séparée ou séquentielle pour le traitement, la prévention et/ou la palliation du cancer

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.04.2010 EP 10290177**

(43) Date of publication of application:
**06.02.2013 Bulletin 2013/06**

(73) Proprietors:
• **Institut Gustave Roussy (IGR)**
**94800 Villejuif (FR)**
• **Université Paris Sud XI**
**91405 Orsay Cedex (FR)**

(72) Inventors:
• **DEUTSCH, Eric**
**F-75004 Paris (FR)**
• **VOZENIN, Marie Catherine**
**F-91580 Villeneuve Sur Auvers (FR)**
• **BOURHIS, Jean**
**F-92330 Sceaux (FR)**

(74) Representative: **Paris, Fabienne et al**
**Ernest Gutmann - Yves Plasseraud S.A.S.**
**3, rue Auber**
**75009 Paris (FR)**

(56) References cited:
**WO-A2-2006/047716**

• **LIMSUKON A ET AL: "Regression of recurrent respiratory papillomatosis with celecoxib and erlotinib combination therapy", CHEST, THE COLLEGE, CHICAGO, IL, US LNKD- DOI: 10.1378/CHEST.08-2639, vol. 136, no. 3, 1 September 2009 (2009-09-01), pages 924-926, XP008125703, ISSN: 0012-3692**
• **LOYO M ET AL: "Aggressive recurrent respiratory papillomatosis in a neonate", INTERNATIONAL JOURNAL OF PEDIATRIC OTORHINOLARYNGOLOGY, ELSEVIER, AMSTERDAM, NL LNKD- DOI:10.1016/J.IJPORL. 2008.02.022, vol. 72, no. 6, 1 June 2008 (2008-06-01), pages 917-920, XP022639209, ISSN: 0165-5876 [retrieved on 2008-04-18]**
• **HERRERA F G ET AL: "Molecular targeted agents combined with chemo-radiation in the treatment of locally advanced cervix cancer", REVIEWS ON RECENT CLINICAL TRIALS, BENTHAM SCIENCE PUBLISHERS, NL LNKD- DOI: 10.2174/157488708784223835, vol. 3, no. 2, 1 May 2008 (2008-05-01), pages 111-120, XP008125405, ISSN: 1574-8871**

**EP 2 552 452 B1**

**(Cont. next page)**

- ANDREI G ET AL: "ANTIPROLIFERATIVE EFFECTS OF ACYCLIC NUCLEOSIDE PHOSPHONATES ON HUMAN PAPILLOMAVIRUS (HPV)-HARBORING CELL LINES COMPARED WITH HPV-NEGATIVE CELL LINES", ONCOLOGY RESEARCH, PERGAMON PRESS, NEW YORK, NY, US, vol. 10, no. 10, 1 January 1998 (1998-01-01), pages 523-531, XP008046881, ISSN: 0965-0407
- ANDREI GRACIELA ET AL: "Induction of apoptosis by cidofovir in human papillomavirus (HPV)-positive cells", 2001, ONCOLOGY RESEARCH, VOL. 12, NR. 9-10, PAGE(S) 397-408, XP008126676, ISSN: 0965-0407 abstract
- HOSTETLER KARL Y ET AL: "Enhanced antiproliferative effects of alkoxyalkyl esters of cidofovir in human cervical cancer cells in vitro", MOLECULAR CANCER THERAPEUTICS, vol. 5, no. 1, January 2006 (2006-01), pages 156-159, XP002599768, ISSN: 1535-7163
- BOSTROM B ET AL: "Gefitinib therapy for life-threatening laryngeal papillomatosis", ARCHIVES OF OTOLARYNGOLOGY HEAD AND NECK SURGERY, AMERICAN MEDICAL ASSOCIATION, US LNKD- DOI: 10.1001/ARCHOTOL.131.1.64, vol. 131, no. 1, 1 January 2005 (2005-01-01), pages 64-67, XP008125409, ISSN: 0886-4470
- FENG WEI ET AL: "Morphoproteomic evidence of constitutively activated and overexpressed mTOR pathway in cervical squamous carcinoma and high grade squamous intraepithelial lesions.", INTERNATIONAL JOURNAL OF CLINICAL AND EXPERIMENTAL PATHOLOGY 2009 LNKD- PUBMED:19079619, vol. 2, no. 3, 2009, pages 249-260, XP002599769, ISSN: 1936-2625

**Description**

## FIELD OF THE INVENTION

The application notably relates to EGFR inhibitor(s) and antiviral agent(s) for simultaneous, separate or sequential use in the treatment and/or prevention and/or palliation of neoplasm(s), more particularly of malignant or premalignant tumor(s), still more particularly of cancer(s), to a combined preparation comprising at least one EGFR inhibitor and at least one antiviral agent, as well as to the medical and biotechnological applications thereof, more particularly in the field of the treatment and/or prevention and/or palliation of neoplasm(s), more particularly of malignant or premalignant tumor(s), still more particularly of cancer(s).

## BACKGROUND OF THE INVENTION

The epidermal growth factor receptor (EGFR) is one of the molecules, which have been identified to be important targets for cancer therapy. EGFR is notably expressed at high levels in a number of tumors, including Squamous Cell Carcinoma of the Head and Neck (SCCHN), cervix, anal and colorectal cancers, where it is associated with poor survival.

The activation of EGFR leads to the activation of intracellular signaling pathways, which are involved in cell proliferation, resistance to apoptosis, and tumor angiogenesis. Therefore, the signaling pathways, which are activated by EGFR, are considered to play a central role in the proliferation and immortalization of cancer cells.

EGFR ligands, which antagonize signal transduction by EGFR, such as monoclonal antibodies that target the extracellular domain of EGFR, or inhibitors of intracellular effector(s) of the EGFR signaling pathway, are currently commercially available.

For example, the anti-EGFR monoclonal antibody cetuximab (Erbitux®) competitively inhibits the binding of EGF and other ligands, such as transforming growth factor alpha (TGF-alpha), to EGFR, thereby antagonizing EGFR signal transduction. Cetuximab (Erbitux®) is indicated in the treatment of locally or regionally advanced SCCHN, recurrent or metastatic SCCHN and metastatic colorectal carcinomas.

Another example is erlotinib (Tarceva®), which is a tyrosine kinase inhibitor that inhibits the intracellular phosphorylation of tyrosine kinase associated with EGFR. Erlotinib (Tarceva®) is indicated in the treatment of locally advanced or metastatic non-small cell lung cancers (NSCLC) and in the treatment of locally advanced or metastatic pancreatic cancers.

Although they express high levels of EGFR, targeted cancer cells do however respond only poorly to. EGFR blockade. The majority of patients do not respond to EGFR inhibitors; and those patients, who are initially responsive, usually develop resistance to EGFR inhibitors This is for example the case with the monoclonal antibody cetuximab (Erbitux®), and with the tyrosine kinase inhibitor erlotinib (Tarceva®), whose respective indications are still limited to treatment in simultaneous or sequential combination with systemic therapy (systemic chemotherapy and/or radiation therapy) and/or to second-intention treatment. Analysis of the factors underlying non-response or resistance to EGFR blockade has revealed an unexpected complexity involving several mechanisms and diverse factors. The current view is that the implementation of several strategies would be required in order to improve efficacy of EGFR inhibitors.

The interest in using EGFR inhibitors is further diminished by the fact that, at the doses at which said EGFR inhibitors are indicated for said treatments, serious adverse reactions, such as severe skin reactions, renal failure, interstitial lung diseases, including fatalities, are frequently observed.

The application provides means, which remove or alleviate the resistance, and/or increase the sensitivity of neoplastic cells to EGFR inhibition. The means of the application notably comprise combining at least one EGFR inhibitor with at least one antiviral agent.

The means of the application are more particularly adapted to virus-positive neoplasms, more particularly to HPV-positive neoplasms.

EGFR inhibitors are currently used in combination with systemic chemotherapy (e.g., in combination with cisplatin or carboplatin) or with radiation therapy, or in monotherapy. (usually as a second-intent treatments). Please see *e.g.,* Vermorken et al. 2008 (Cancer 112: 2710-2719), describing therapeutic tests using the EGFR inhibitor cetuximab (Erbitux®) in combination with cisplatin.

It is also known that antiviral agents can be advantageously combined with radiation therapy. Please see *e.g.,* WO 01/32215 Al; Abdulkarim *et al.* 2002; Amine *et al.* 2006.

WO 01/32215 Al notably discloses that an antiviral nucleoside phosphanate analogue, such as cidofovir (Vistide®), induces a marked increase in radiosensitization in cancer cells, more particularly in virus-associated cancer cells, such as HPV-associated cancer cells.

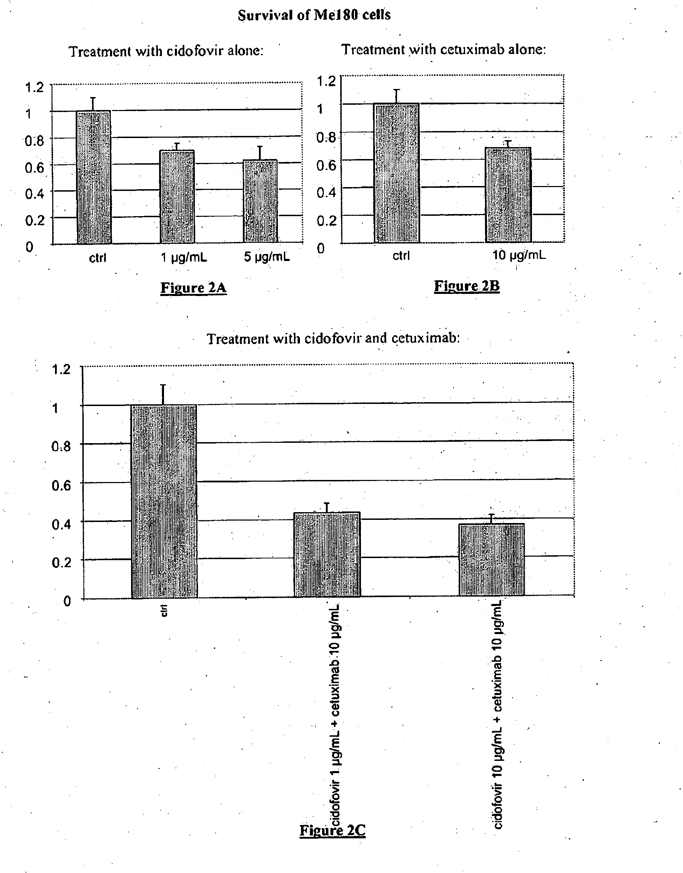

Abdulkarim *et al.* 2002 discloses that the increased radiosensitivity that is obtained when combining cidofovir with irradiation notably results from the fact that irradiation induces the up-regulation of HPV E6/E7 proteins, and that cidofovir prevents or inhibits this undesired E6/E7 up-regulation (please see e.g., paragraph bridging pages 2341-2342 and Figure 2 of Abdulkarim *et al.* 2002).

Amine *et al.* 2006 that discloses the cidofovir-induced E6 inhibition is followed by an anti-VEGF effect on HPV18-positive cell lines.

It is however believed that the prior art does not disclose combining an EGFR inhibitor with an antiviral agent. It is further believed that, contrary to what has been disclosed for irradiation, the prior art does not disclose or suggest that EGFR inhibitors would be beneficial if combined with an antiviral agent.

It is also believed that the prior art does not suggest that an antiviral agent potentiates the EGFR blockade induced by EGFR inhibitor, more particularly that an antiviral agent removes or alleviates the resistance, and/or increases the sensitivity of neoplastic cells to EGFR inhibition.

It is furthermore believed that decreasing the virus load, more particularly the HPV load, of EGFR-positive cancer patients, was not an obvious trend in the prior art, since it had been observed that if a patient's tumor had high EGFR expression and contained HPV, the survival strategy was improved compared to those without HPV (*cf. e.g.,* Kumar *et al.* 2008).

## SUMMARY OF THE INVENTION

The present invention is defined in the appended claims.

Subject matters which are not encompassed by the scope of the claims do not form part of the present invention.

The application notably relates to a combination or association of compounds (or of biologically active compounds, or of therapeutic agents), and to a combined preparation, preferably a combined pharmaceutical preparation. The combination, association or combined preparation of the application comprises at least one antiviral agent and at least one EGFR inhibitor for simultaneous, separate or sequential use or administration in a subject, more particularly in an animal (still more particularly a mammal, advantageously a human). The application also relates to the medical and biotechnological applications of said combination, association or combined preparation, more particularly in the field of neoplasm treatment and/or prevention and/or palliation.

The combination, association, or combined preparation of the application shows a synergistic effect on neoplastic cells. The antiviral agent comprised in the combination, association or combined preparation of the application potentiates the EGF blockade induced by the EGFR inhibitor. More particularly, the antiviral agent removes or alleviates the resistance, and/or increases the sensitivity of neoplastic cells to EGFR inhibition. The combination, association or combined preparation of the application enables to achieve an unexpected decrease in the level of surviving neoplastic cells.

The inventors demonstrate that the means of the application increase the response of neoplastic cells, more particularly of malignant or pre-malignant tumor cells, to EGFR inhibitor(s) in a synergistic manner. Said synergy has been observed for virus-positive neoplastic cells, more particularly for HPV-positive neoplastic cells, as well as for virus-negative, more particularly HPV-negative cells.

The magnitude of the synergy between the antiviral agent and the EGFR inhibitor is markedly dependent upon HPV status, the most important effects being observed in HPV-positive neoplastic cells.

The inventors have more particularly discovered that, when the means of the application are applied to HPV-positive neoplastic cells, said synergistic effect is observed at doses of antiviral agent, more particularly of antiviral agent and EGFR inhibitor, lower than expected, which is especially advantageous since the risk of adverse reactions that may be caused by each of the two drugs, more particularly by the antiviral agent (renal toxicity) then is very limited.

Said synergistic effect (including the low dose surprising effect observed on HPV-positive neoplastic cells) is herein demonstrated for antiviral nucleoside phosphonate analogues, such as cidofovir (Vistide®), and for antiviral pyrophosphate analogues, such as foscarnet (Foscarvir®).

The means of the application or invention are more particularly indicated for those subjects, who are non-responsive, or have become resistant to, a systemic monotherapy by cisplatin, or for whom a systemic monotherapy by cisplatin is contraindicated.

The means of the application or invention are more particularly indicated for the treatment and/or prevention and/or palliation of Squamous Cell Carcinoma of the Head and Neck (SCCHN), recurrent and/or metastatic SCCHN, cervix tumor(s), vulvar tumor(s), penile tumor(s), anal tumor(s), colorectal tumor(s), lung tumor(s), Non-Small Cell Lung Cancer (NSCLC), locally advanced or metastatic NSCLC; and nasopharyngeal tumor(s).

The invention relates more particularly to a combination of biologically active compounds comprising at least one antiviral agent and at least one EGFR inhibitor, for simultaneous, separate or sequential use in the treatment and/or prevention and/or palliation of malignant or pre-malignant neoplasms,

wherein said at least one antiviral agent is cidofovir, foscarnet or a salt thereof,

wherein said at least one EGFR inhibitor comprises cetuximab, erlotinib or a salt thereof,

wherein said neoplasms are Squamous Cell Carcinoma of the Head and Neck (SCCHN), recurrent and/or metastatic SCCHN, cervix tumor(s), vulvar tumor(s), penile tumor(s), anal tumor(s), colorectal tumor(s), lung tumor(s), Non-Small Cell Lung Cancer (NSCLC), locally advanced or metastatic NSCLC, nasopharyngeal tumor(s), and wherein said neoplasms are:

- *Papillomaviridae*-positive and/or EBV-positive, or are
- virus-negative and not virus-associated.

## BRIEF DESCRIPTION OF THE FIGURES

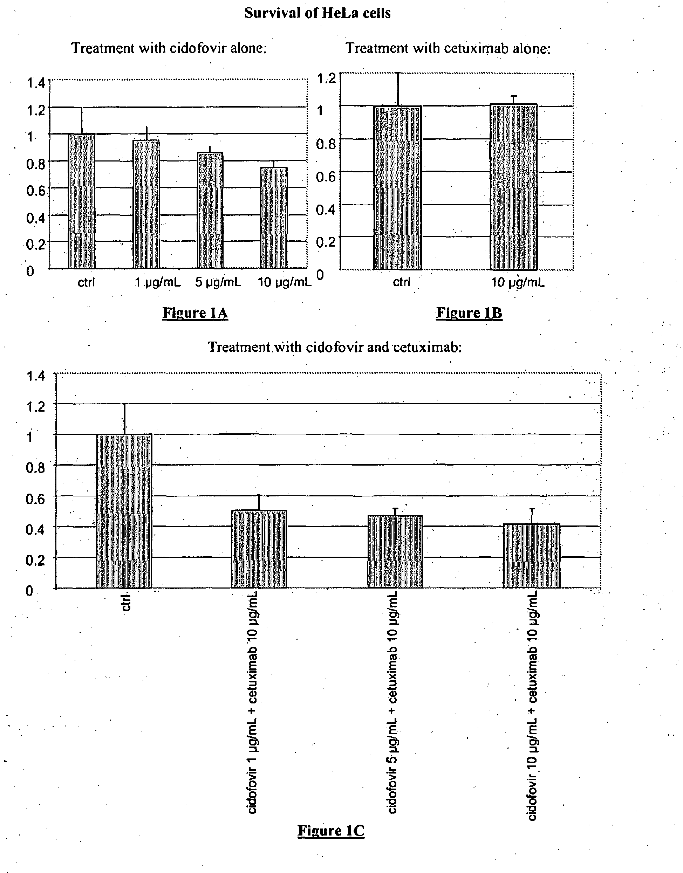

Figures 1A, 1B and 1C illustrate the cell survival (clonogenicity) of HeLa cells (human HPV-positive cervical carcinoma cells) after treatment with cidofovir (Vistide®) alone, or with cetuximab (Erbitux®) alone, or with a combination of the two drugs, respectively (ctrl = control treatment without cidofovir and without cetuximab).
Figure 1A illustrates the survival of HeLa cells after treatment with cidofovir alone at a concentration of 1, 5 or 10 $\mu$g/mL.
Figure 1B illustrates the survival of HeLa cells after treatment with cetuximab alone at a concentration of 10 $\mu$g/mL.
Figure 1C illustrates the survival of HeLa cells after treatment with both cidofovir and cetuximab (cidofovir 1, 5 or 10 $\mu$g/mL and cetuximab 10 $\mu$g/mL).
Figures 2A, 2B and 2C illustrate the cell survival (clonogenicity) of Me180 cells (human HPV-positive cervical carcinoma cells) after treatment with cidofovir (Vistide®) alone, or after treatment with cetuximab (Erbitux®) alone, or after treatment with a combination of the two drugs, respectively (ctrl = control treatment without cidofovir and without cetuximab).
Figure 2A illustrates the survival of Me180 cells after treatment with cidofovir alone at a concentration of 1 or 5 $\mu$g/mL.
Figure 2B illustrates the survival of Me180 cells after treatment with cetuximab alone at a concentration of 10 $\mu$g/mL.
Figure 2C illustrates the survival of Me180 cells after treatment with both cidofovir and cetuximab (cidofovir 1 or 10 $\mu$g/mL and cetuximab 10 $\mu$g/mL).

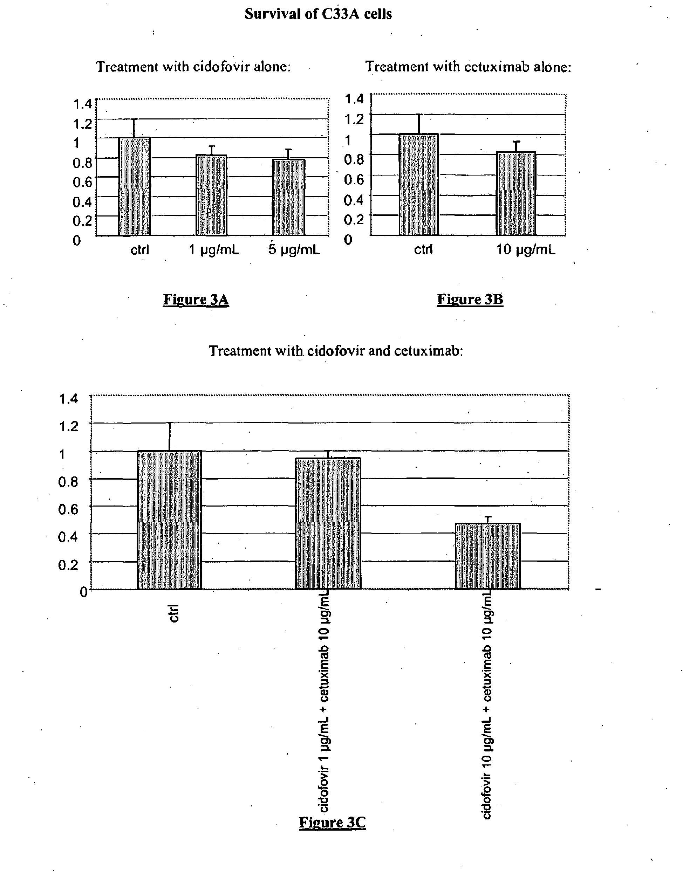

Figures 3A, 3B and 3C illustrate the survival (clonogenicity) of C33A cells (human HPV-negative cervical carcinoma cells) after treatment with cidofovir (Vistide®) alone, or after treatment with cetuximab (Erbitux®) alone, or after treatment with a combination of the two drugs, respectively (ctrl = control without cidofovir and without cetuximab).
Figure 3A illustrates the survival of C33A cells after treatment with cidofovir alone at a concentration of 1 or 5 $\mu$g/mL.
Figure 3B illustrates the survival of C33A cells after treatment with cetuximab alone at a concentration of 10 $\mu$g/mL,
Figure 3C illustrates the survival of C33A cells after treatment with both cidofovir and cetuximab (cidofovir 1 or 10 $\mu$g/mL and cetuximab 10 $\mu$g/mL).

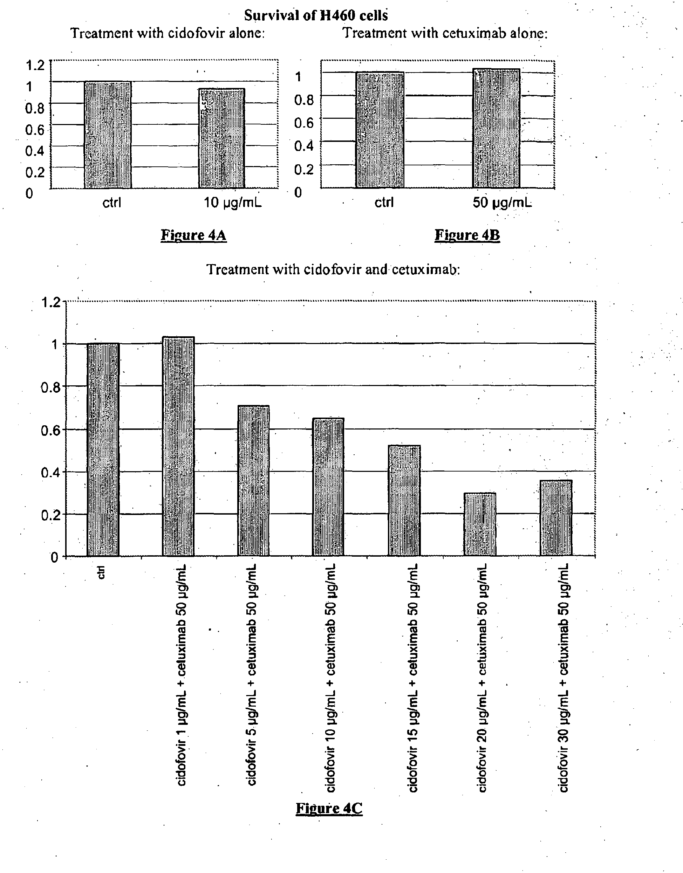

Figures 4A, 4B and 4C illustrate the survival (clonogenicity) of H460 cells (human HPV-negative cervical carcinoma cells) after treatment with cidofovir (Vistide®) alone, or after treatment with cetuximab (Erbitux®) alone, or after treatment with a combination of the two drugs, respectively (ctrl = control without cidofovir and without cetuximab).
Figure 4A illustrates the survival of H460 cells after treatment with cidofovir alone at a concentration of 10 $\mu$g/mL.
Figure 4B illustrates the survival of H460 cells after treatment with cetuximab alone at a concentration of 50 $\mu$g/mL.
Figure 4C illustrates the survival of H460 cells after treatment with both cidofovir and cetuximab (cidofovir 1, 5, 10, 15, 20 or 30 $\mu$g/mL and cetuximab 50 $\mu$g/mL).

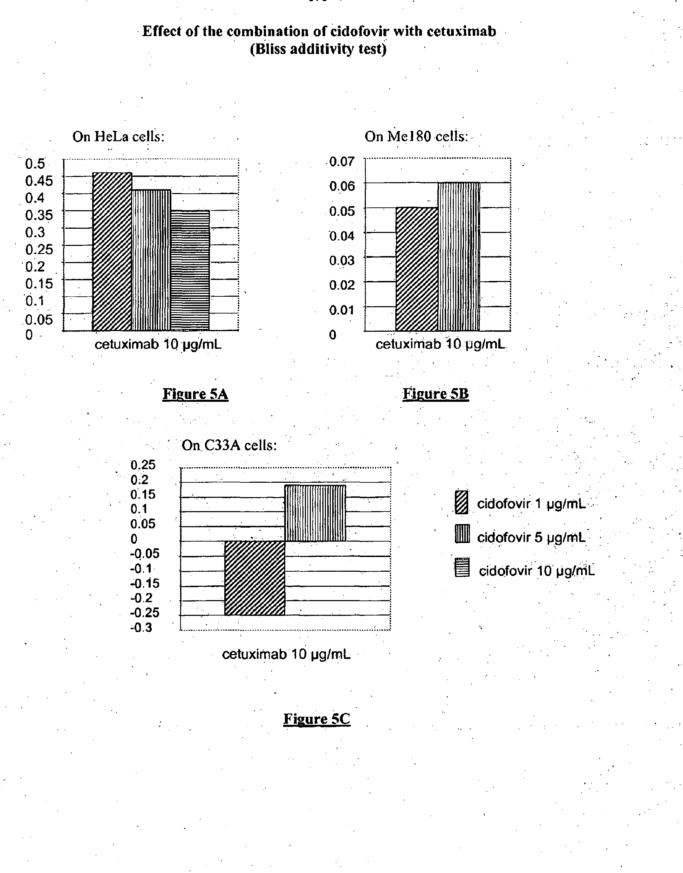

Figures 5A, 5B and 5C illustrate the results of the Bliss additivity test for the combination of cidofovir (Vistide®) and cetuximab (Erbitux®) on HeLa cells, Me180 cells and C33A cells, respectively, *i.e.,* on two human HPV-positive cervical carcinoma cell lines (HeLa cells in Figure 5A, Me180 cells in Figure 5B) and on one human HPV-negative cervical carcinoma cell line (C33A cells in Figure 5C).
Figure 5A (HeLa cells), from left to right:

- cidofovir 1 $\mu$g/mL + cetuximab 10 $\mu$g/mL;
- cidofovir 5 $\mu$g/mL+cetuximab 10 $\mu$g/mL;
- cidofovir 10 $\mu$g/mL + cetuximab 10 $\mu$g/mL;

Figure 5B (Me180 cells), from left to right:

- cidofovir 1 $\mu$g/mL + cetuximab 10 $\mu$g/mL;
- cidofovir 5 $\mu$g/mL + cetuximab 10 $\mu$g/mL;

Figure 5C (C33A cells), from left to right:

- cidofovir 1 $\mu$g/mL + cetuximab 10 $\mu$g/mL;

- cidofovir 5 $\mu$g/mL + cetuximab 10 $\mu$g/mL.

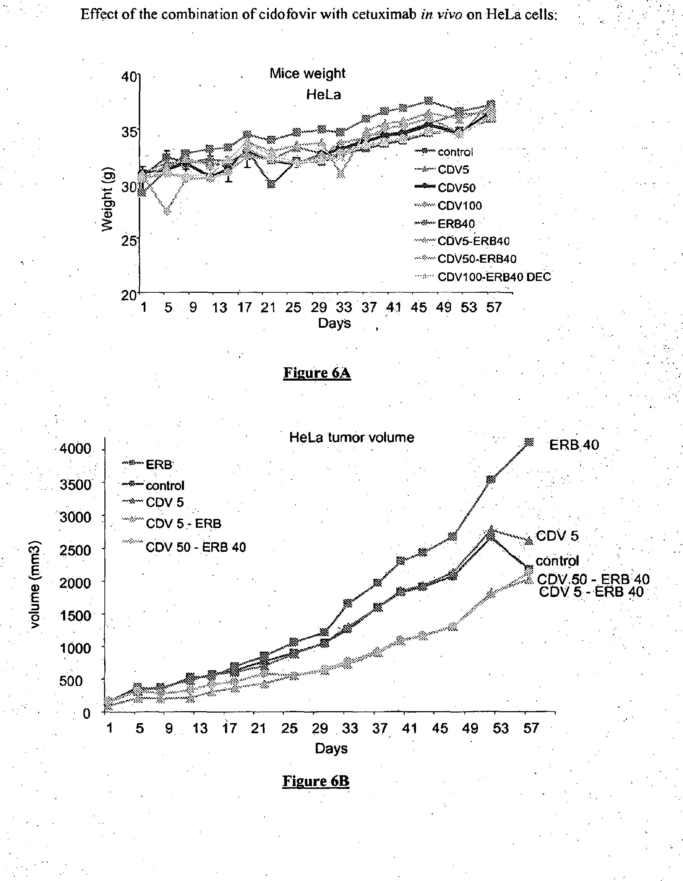

Figure 6A and 6B illustrate the efficacy of the cidofovir and cetuximab combination in *vivo* in HeLa xenograft.
Figure 6A illustrates the mice weight variation. First injection of-drugs in mice causes a small weight loss during the week of treatment, but then the weight is back and constant.
Figure 6B illustrates the tumor volume variation. Regarding the evolution of tumor volume, the injection of cetuximab alone or of cifovir alone does not reduce tumor growth compared to the control group, whereas mice injected with cidofovir in combination with cetuximab showed delayed tumor growth compared to the control group.

CDV = cidofovir (Vistide®)
ERB = cetuximab (Erbitux®)
CDV50 = cidofovir 50 $\mu$g/mL
ERB 40 = cetuximab 40 $\mu$g/mL
CDV50-ERB40 = cidofovir 50 $\mu$g/mL and cetuximab 40 $\mu$g/mL

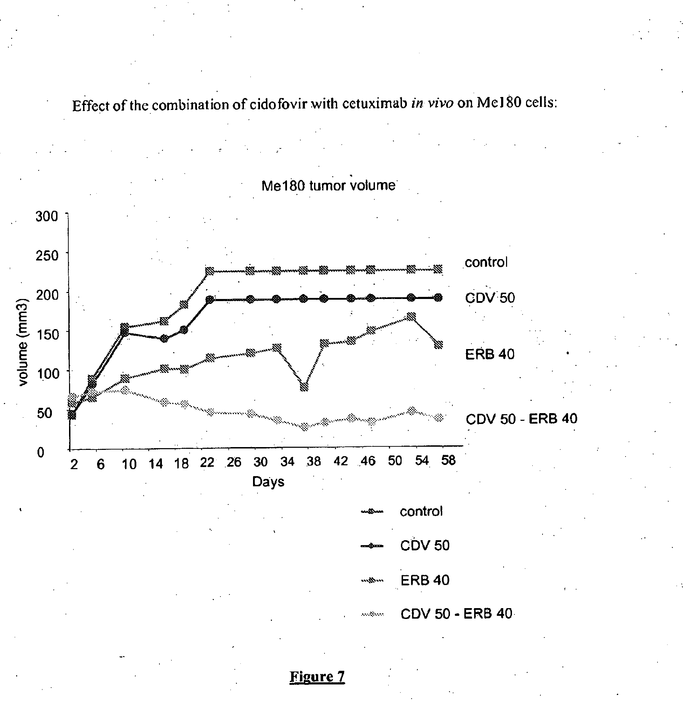

Figure 7 illustrates the efficacy of the cidofovir and cetuximab combination *in vivo* in Me180 xenograft. Cidofovir alone does not reduce tumor growth compared to the control group, whereas mice injected with cetuximab alone and mice injected with cidofovir in combination with cetuximab showed delayed tumor growth compared to the control group. The delay effect is more particularly pronounced for those mice, which have received the drug combination.

CDV = cidofovir (Vistide®)
ERB = cetuximab (Erbitux®)
CDV 5 = cidofovir 5 $\mu$g/mL
CDV50 = cidofovir 50 $\mu$g/mL
CDV100 = cidofovir 100 $\mu$g/mL
ERB 40 = cetuximab 40 $\mu$g/mL
CDV5-ERB40 = cidofovir 5 $\mu$g/mL and cetuximab 40 $\mu$g/mL
CDV50-ERB40 = cidofovir 50 $\mu$g/mL and cetuximab 40 $\mu$g/mL
CDV100-ERB40 DEC = cidofovir 100 $\mu$g/mL and cetuximab 40 $\mu$g/mL

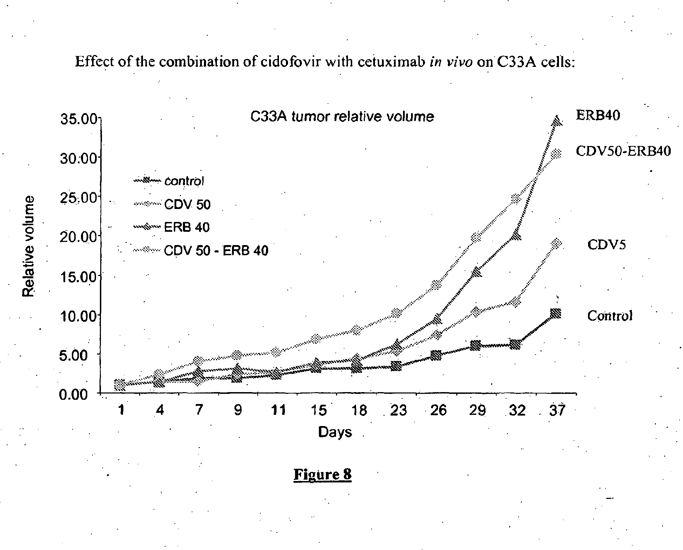

Figure 8 illustrates the efficacy of the drug combination *in vivo* in C33A xenograft.
No synergy of the drug combination has been observed *in vivo* with the HPV-negative C33 cell line.

CDV = cidofovir (Vistide®)
ERB = cetuximab (Erbitux®)
CDV50 = cidofovir 50 $\mu$g/mL
ERB 40 = cetuximab 40 $\mu$g/mL
CDV50-ERB40 = cidofovir 50 $\mu$g/mL and cetuximab 40 $\mu$g/mL

## DETAILED DESCRIPTION OF THE INVENTION

The inventors have notably found that antiviral agents, such as cidofovir (e.g., Vistide®) or foscarnet *(e.g., Foscarvir®)*, potentiate the killing of neoplastic cells induced by EGFR inhibitors, such as cetuximab *(e.g.,* Erbitux®) or erlotinib *(e.g.,* Tarceva®), and that this potentiation is exerted in a synergistic manner.
For example, the clonogenic survival of Human Papilloma Virus positive (HPV-positive) neoplastic cells after exposure to cidofovir alone at 1 $\mu$g/mL, cetuximab alone at 10 $\mu$g/mL or the combinaison of both is about 100%, 100% and 50% on HeLa cells, respectively (*cf.* Figure 1C).
The application or invention enables using doses of EGFR inhibitor(s), which are lower than expected (e.g., in view of the doses of EGFR inhibitor, which are currently used in the prior art EGFR inhibitor treatments).
Said synergy was observed on HPV-positive neoplastic cells, as well as on HOV-negative, more particularly virus-negative, neoplastic cells.
The inventors further demonstrate that, when the administration of the antiviral agent is associated with the administration of an EGFR inhibitor, the synergistic effect(s) exerted by the antiviral agent on HPV-positive neoplastic cells, occur at relatively low doses, *i.e.,* at doses that are largely below the plasma levels achieved after administration of the dose of the antiviral agent that is currently approved for its antiviral applications. For example, a synergistic effect is observed on HPV-positive neoplastic cells treated with an association of cidofovir at 1 $\mu$g/mL and of cetuximab at 10 $\mu$g/mL (*cf.* Figures 1C and 2C), whereas the plasmatic concentration of cidofovir applied at 3 mg/kg for the treatment of CMV retinitis in AIDS patients (*i.e.,* the currently approved clinical application of cidofovir) is 7.6 $\pm$ 1.4 $\mu$g/mL after 1 hour of cidofovir infusion.

Hence, at least with HPV-positive neoplastic cells, the application or invention enables administering not only lower doses of EGFR inhibitor(s) than expected, but also lower doses of antiviral agent(s) than expected, which is especially advantageous, since many antiviral agents (such as cidofovir) are highly toxic, more particularly at the renal level. On HPV-negative neoplastic cells, more particularly on virus-negative neoplastic cells, said synergistic effect is also observed, but at higher doses of antiviral agent(s) and/or EGFR inhibitor(s) compared to HPV-positive neoplastic cells (*cf.* Figures 3C and 4C). When used on HPV-negative neoplastic cells, more particularly on virus-negative neoplastic cells, the application or invention nonetheless still has the advantage of enabling the administration of lower doses of EGFR inhibitor(s) than expected.

The inventors further bring the demonstration that the effect(s) observed *in vitro* (*cf.* example 1) are confirmed *in vivo* (*cf.* examples 2 and 3).

The application thus relates to the association, or the combination, of at least one antiviral agent (preferably at least one anti-HPV agent) and of at least one EGFR inhibitor for simultaneous, separate or sequential use in a medical treatment and/or prevention and/or palliation, and/or for simultaneous, separate or sequential administration to a subject in need thereof (more particularly to an animal in need thereof, still more particularly to a mammal in need thereof, advantageously to a human in need thereof).

Advantageously, said use or administration is intended for the treatment and/or prevention and/or palliation of neoplasm(s), more particularly of virus-positive neoplasm(s) or virus-negative neoplasm(s), still more particularly of virus-positive neoplasm(s), advantageously of HPV-positive neoplasm(s)).

The invention relates more particularly to a combination of biologically active compounds comprising at least one antiviral agent and at least one EGFR inhibitor, for simultaneous, separate or sequential use in the treatment and/or prevention and/or palliation of malignant or pre-malignant neoplasms,
wherein said at least one antiviral agent is cidofovir, foscarnet or a salt thereof,
wherein said at least one EGFR inhibitor comprises cetuximab, erlotinib or a salt thereof,
wherein said neoplasms are Squamous Cell Carcinoma of the Head and Neck (SCCHN), recurrent and/or metastatic SCCHN, cervix tumor(s), vulvar tumor(s), penile tumor(s), anal tumor(s), colorectal tumor(s), lung tumor(s), Non-Small Cell. Lung Cancer (NSCLC), locally advanced or metastatic NSCLC, nasopharyngeal tumor(s), and
wherein said neoplasms are:

- *Papillomaviridae-positive* and/or EBV-positive, or are
- virus-negative and not virus-associated.

Said at least one antiviral agent and said at least one EGFR inhibitor can be physically distinct, at least to a sufficient extent for each of the two products to be separately or sequentially administrable. They may for example be associated or aggregated together as distinct physical units contained in a kit-of-parts, collection or package.

When said at least one antiviral agent and said at least one EGFR inhibitor are intended for simultaneous use, they can either be physically distinct or be in intimate contact or association. In the latter case, they may for example be mixed together, and/or directly or indirectly complexed onto each other, and/or directly or indirectly linked to each other (*e.g.,* by covalent linkage). Such a mixture, complex or linked product is herein encompassed by the application or invention.

Whichever physical form the association or combination takes, said at least one antiviral agent and said at least one EGFR inhibitor should be in a condition, where they can be administered to a subject so as to functionality cooperate and thereby exert the synergistic effect of the application or invention.

Therefore, the application or invention notably relates to:

- a combination or association of compounds, or of biologically active compounds, or of therapeutic agents, wherein said combination or association comprises said at least one antiviral agent and said at least one EGFR inhibitor for simultaneous, separate or sequential use or administration in a subject, more particularly in an animal (still more particularly a mammal, advantageously a human);
- a combined preparation, preferably a combined pharmaceutical preparation, comprising said at least one antiviral agent and said at least one EGFR inhibitor for simultaneous, separate or sequential use or administration in a subject, more particularly in an animal (still more particularly a mammal, advantageously a human);

as well as to the medical and biotechnological applications thereof, more particularly in the field of neoplasm treatment and/or prevention and/or palliation, including methods for the treatment and/or prevention and/or palliation of neoplasm(s) or tumor(s), comprising administering a combination or combined preparation of the application or invention (more particularly a therapeutically effective amount thereof) to a subject in need thereof

A combined preparation of the application or invention includes preparations, where said at least one antiviral agent and said at least one EGFR inhibitor are physically sufficiently distinct for being separately or sequentially administrable. Of course, such a combined preparation can also be used for simultaneous administration.

A combined preparation of the application or invention also includes preparations, where said at least one antiviral agent and said at least one EGFR inhibitor are in intimate contact or association with each other (e.g., compositions comprising said at least one antiviral agent and said at least one EGFR inhibitor in mixture), whereby only simultaneous administration can then be contemplated.

A combination or combined preparation of the application or invention can optionally comprise one or more pharmaceutically acceptable carriers or excipients.

A combination or combined preparation of the application can optionally comprise one or more active ingredients other than antiviral agents and EGFR inhibitors, e.g., one or more other anti-neoplastic products.

In the application, unless specified otherwise or unless a context dictates otherwise, all the terms have their ordinary meaning in the relevant field(s).

For example, the term "neoplastic cell(s)" is to be understood in accordance with its ordinary meaning in the field of tumor development.

The term "neoplastic cell(s)" refers to any kind of tumor cells(s), *i.e.,* benign, pre-malignant and malignant tumor cell(s). It refers more particularly to pre-malignant and malignant tumor cell(s), still more particularly to malignant cell(s). (i.e., cancer cell(s)). Similarly, the term "neoplasm(s)" refers to benign, pre-malignant and malignant tumor(s), more particularly to pre-malignant and malignant tumor(s), still more particularly to malignant tumor(s) (i.e., cancer(s)).

The term "pre-malignant tumor(s)" refers to refers to tumor(s) which, if left untreated, may lead to malignant tumor(s).

### 1. Antiviral agent(s):

The term "antiviral agent(s)" is to be understood in accordance with its ordinary meaning in the field of the treatment and/or prevention and/or palliation of viral infection(s).

The term "antiviral agent(s)" refers to a product, more particularly to a product comprising one or several active principle(s) selected from those compounds, molecules, complexes and compositions, which can be administered to animals, more particularly to mammals, still more particularly to humans, as a medication, and which exert an antiviral effect, *i.e.,* which inhibit or block the development of at least one virus in said animals, mammals or humans.

Hence, an antiviral agent inhibits or blocks the development of at least one virus in an animal, more particularly in a mammal, still more particularly in a human, in accordance with a risk/benefit ratio that is favorable to the health of said animal, mammal or human.

In the application or invention, preferred viruses are those which are associated, with, or which induce, a neoplasm in an animal, more particularly a mammal, still more particularly a human.

In the application or invention, said antiviral effect preferably is specific of a virus type, family, sub-family, genus or species.

According to a particular embodiment of the application, said antiviral effect is shown against DNA virus(es). According to an aspect of the application or invention, said antiviral effect is shown against DNA virus(es), without substantially inhibiting or blocking the development of retroviruses. According to a particular aspect of the application or invention, said anti-DNA virus effect is specifically shown against DNA virus(es).

In the application or invention, DNA viruses are viruses, which have DNA as its genetic material and replicate using a DNA-dependent DNA polymerase, and which belong, to either Group I (double-stranded DNA viruses) or Group II (single-stranded DNA viruses) of the Baltimore classification system for viruses.

Illustrative DNA viruses in accordance with the application comprise Group I viruses, such as:

- viruses of the Order *Caudovirales,* more particularly of the *Myoviridae, Podoviridae* or *Siphoviridae* family,
- viruses of the Order *Herpesvirales,* more particularly of the *Alloherpesviridae, Herpesviridae* (which includes the Epstein Barr Virus (EBV)) or *Malacoherpesvindae* family,
- viruses of unassigned family or genus, more particularly viruses of the *Ascoviridae, Adenoviridae, Asfarviridae, Baculoviridae, Coccolithoviridae, Corticoviridae, Fuselloviridae, Guttaviridae, Iridoviridae, Lipothrixviridae, Mimiviridae, Nimaviridae, Papillomaviridae* (which includes HPV), *Phycodnaviridae, Plasmaviridae, Polyomaviridae, Poxviridae, Rudiviridae* or *Tectiviridae* family, and viruses of the *Ampullavirus, Nudivirus, Salterprovirus, Sputnik virophage* or *Rhizidiovirus* genus. Illustrative DNA viruses in' accordance with the application comprise Group II viruses, such as viruses of the *Inoviridae, Microviridae, Anelloviridae, Circoviridae, Geminiviridae, Nanoviridae* or *Parvoviridae family.*

According to an embodiment of the application, DNA viruses in accordance with the application or invention comprise Group I viruses, more particularly viruses of the *Herpesviridae* family, such as EBV, and of the *Papillomaviridae* family, such as HPV, more particularly the so-called High Risk (HR) HPV, such as HPV16 and HPV18. According to a particular embodiment of the application or invention, DNA viruses in accordance with the application or invention comprise viruses of the *Papillomaviridae* family, advantageously HPV, more particularly the so-called High Risk (HR) HPV, such as HPV16, HPV18, HPV31 and HPV45, more particularly HPV16 and HPV18.

In the application, said antiviral effect can be exerted e.g., by inhibiting or blocking the viral nucleic acid chain elongation (of the viral DNA chain in the case of DNA viruses), e.g., by inhibiting or blocking the incorporation of natural deoxynucleotide(s) into the growing viral nucleic acid chain.

Such an inhibition or blockade can be exerted e.g.,:

- by directly competing with natural deoxynucleotide(s) for incorporation into the growing viral nucleic acid chain (e.g., such as.nucleic acid analogues do), and/or
- by inhibiting or blocking the function(s) of the viral polymerase (of the DNA-dependent DNA polymerase in the case of DNA viruses) and/or of protein(s) and/or enzyme(s) involved in the natural functioning of the viral polymerase, e.g., by binding on a polymerase sub-unit or site involved in the proper functioning of said viral polymerase (e.g., such as pyrophosphate analogues do)

In an embodiment of the application, said antiviral agents comprise:

- nucleic acid analogues (*i.e.,* structural mimics of natural deoxynucleotide(s), which compete with natural deoxynucleotide(s) for incorporation into the growing viral nucleic acid chain), and
- pyrophosphate analogues (*i.e.,* structural mimics of the anion pyrophosphate, which inhibit or block the function(s) of the viral polymerase and/or of protein(s) and/or enzyme(s) involved in the natural functioning of the viral polymerase), and
- salts thereof.

Nucleic acid analogues notably comprise:

- nucleobase analogues (preferably, analogues of pyrimidine-derived nucleobase or analogues of purine-derived nucleobase, more preferably analogues of natural bases, *i.e.,* analogues of guanine, adenine, cytosine or thymine) and saltsthereof, and
- related analogues, whose structure can be considered to be deriving from the structure of a nucleobase analogue, e.g., by addition of one or more groups, such as addition of a sugar (sugar ring or acyclic sugar) and/or of one to three phosphate or phosphonate groups, such as:

    o nucleoside analogues (whose structure can be seen as the one of a nucleobase analogue but with a sugar ring),
    o acyclic nucleoside analogues (whose structure can be seen as the one of an nucleoside analogue, wherein the nucleoside sugar ring has been replaced by an open-chain carbohydrate structure),
    ∘ acyclic nucleoside phosphonate analogues (whose structure can be seen as the one of an acyclic nucleoside analogue further comprising a phosphonate group),
    ∘ nucleotide analogues (whose structure can be seen as the one of a nucleobase analogue but with a sugar ring and three phosphonate groups), and
    ∘ salts thereof.

In an embodiment of the application, said nucleic acid analogues comprise acyclic nucleoside phosphonate analogues (such as cidofovir, *e.g.,* commercialized under Vistide®) and acyclic nucleoside analogues (such as acycloguanosine, e.g., commercialized under Aciclovir®), advantageously acyclic nucleoside phosphonate analogues (such as cidofovir), and salts thereof.

In an embodiment of the application, said pyrophosphate analogues comprise foscarnet (e.g., foscarnet sodium commercialized under Foscarvir®), and salts thereof.

Hence, in an aspect of the application, said antiviral agents--comprise acyclic nucleoside phosphonate analogues (more particularly cidofovir, such as the cidofovir form commercialized under Vistide®), acyclic nucleoside analogues (more particularly acycloguanosine, such as the acycloguanosine form commercialized under Aciclovir®), and pyrophosphate analogues (more particularly foscarnet, such as the foscarnet sodium form commercialized under Foscarvir®), and salts thereof.

In a particular aspect of the application, said antiviral agents comprise acyclic nucleoside phosphonate analogues (more particularly cidofovir, such as the cidofovir form commercialized under Vistide®), and pyrophosphate analogues (more particularly foscarnet, such as the foscarnet sodium form commercialized under Foscarvir®); and salts thereof.

In an embodiment of the application, said antiviral agent is active against DNA virus(es).

In a particular embodiment of the application, said antiviral agent is a product, which has an antiviral effect against DNA virus(es), without having a substantial antiviral effect against retroviruses. In a. particular embodiment of the application, said antiviral agent is not a reverse transcriptase inhibitor. In a particular embodiment of the application or invention, said antiviral effect is specifically exerted against DNA virus(es).

Advantageously, said DNA viruse(s) is(are) HPV and/or EBV, preferably HPV, more preferably HR HPV, such as HPV16 and/or HPV18 and/or HPV31 and/or HPV45, more particularly HPV16 and/or HPV18.

Said antiviral agent advantageously is in a form administrable to an animal, more particularly to a mammal, advantageously to a human. The form in which said antiviral agent is administered to said animal, mammal or human can comprise the antiviral active principle(s), and/or a salt thereof (such as a sodium salt thereof).

Said antiviral agent can be in any administration form, which is found suitable by the person of ordinary skill in the art.

For example, said antiviral agent can be in a form suitable for oral administration (such as a liquid or a solid), for sublingual administration, for inhalation, for insufflation, for injection (*e.g.*, for intramuscular, intravenous, intraperitoneal, intraosseous administration), for rectal administration (e.g., as a suppository), for vaginal administration (e.g., as a suppository).

In an embodiment of the application or invention, said forms of administration comprise injection, more particularly intravenous injection.

According to an aspect of the application or invention, said at least one antiviral agent is cidofovir (or a salt thereof), which is an acyclic nucleoside phosphonate analogue.

According to another aspect of the application or invention, said at least one antiviral agent is acycloguanosine (or a salt thereof), which is an acyclic nucleoside analogue (i.e., an acyclic guanosine analogue).

According to another aspect of the application or invention, said at least one antiviral agent is foscarnet (or a salt thereof), which is a pyrophosphate analogue.

Any combination of two or more of said antiviral agents, which is found to be appropriate by the person of ordinary skill in the art, is also herein encompassed.

In an embodiment of the application or invention, said at least one antiviral agent is cidofovir (Vistide®), or acycloguanosine (Aciclovir®), or foscarnet (Foscarvir®), or a salt thereof, advantageously cidofovir (Vistide®) or foscarnet (Foscarvir®) or a salt thereof, more particularly cidofovir (Vistide®) or a salt thereof.


1.1. Cidofovir (e.g., Vistide®)


Cidofovir is 1-[(S)-3-hydroxy-2-(phophonomethoxy)propyl]cytosine dehydrate.(HPIvIPC), with the molecular formula of $C_8H_{14}N_3O_6P \cdot 2H_2O$ and a MW of 315.22 (279.19 for anhydrous). It is an acyclic nucleoside phosphonate analogue.

Cidofovir enters cells by fluid-phase endocytosis and is phosphorylated to cidofovir monophosphate and subsequently to cidofovir diphosphate, which is believed to be the active intracellular metabolite of cidofovir.

Cidofovir is known to inhibit or suppress cytomegalovirus (CMV) replication by selective inhibition of viral DNA synthesis. Cidofovir is also known to be active *in vitro* against a variety of isolates of CMV and other herpesviruses such as Herpes Simplex Virus (HSV) type 1 or type 2 (HSV-1; HSV-2). Cidofovir diphosphate inhibits herpesvirus polymerases at concentrations that are 8- to 600-fold lower than those needed to inhibit human cellular DNA polymerases.

Cidofovir is currently commercialized under the trademark Vistide®, and has been clinically approved for the treatment of CMV retinitis in patients with acquired immunodeficiency syndrome (AIDS).

Some research studies have reported that cidofovir might have some efficacy in the treatment of acycloguanosine resistant herpes.

Cidofovir is currently being investigated as a complementary intralesional therapy against recurrent respiratory papillomatosis caused by HPV. Please see e.g., Broekema and Dikkers 2008.

Some other research studies have also suggested that cidofovir would be a potential antitumor agent due to its suppression of basic fibroblast growth factor (FGF2). Please see *e.g.,* Liekens 2008.

Vistide® is manufactured by Ben Venue Laboratories, Inc. (Bedford, OH 44146-0568; U.S.A.), and is manufactured for and distributed by Gilead Sciences, Inc. (Foster City, CA 94404; U.S.A.). Vistide® is supplied as an injectable solution containing 375mg of anhydrous cidofovir in 5mL aqueous solution at a concentration of 75 mg/mL (pH 7.4). Vistide® is covered by US patent 5,142,051, the US child application(s) and patent(s) thereof, as well as by,the non-US counterpart patents and patent applications thereof.

For its clinically approved application, *i.e.,* for the treatment of;CMV retinitis in AIDS patients, the approved dose of Vistide® is 5 mg/kg body weight (at first, induction dose of 5mg/kg once per week for two consecutive weeks; and then a maintenance dose of 5mg/kg once every two weeks).

At such a regimen, Vistide® exerts various toxicities. Adverse reactions that are commonly associated with the administration of Vistide® in the treatment of CMV retinitis in AIDS patients notably include blood and lymphatic system disorders, *e.g.,* neutropenia; nervous system disorders, *e.g.,* headache; eye disorders, *e.g.,* iritis, uveitis, hypotony of the eye; respiratory, thorasic and mediastinal disorders, *e.g.,* dyspnea; gastrointestinal disorders, *e.g.,* nausea, vomiting; skin and subcutaneous tissue disorders, *e.g.,* alopecia, rash; renal and urinary disorders, *e.g.,* increase in urine protein level (proteinuria), increase of blood creatine (creatinemia).

The CMV patient must be administered with probenecid (Benemide®) and must be intravenously hydrated before and after administration of Vistide® so as to limit Vistide® renal toxicity.

Reports of renal failure (and of events possibly caused by renal failure, *e.g.,* increase of blood creatine, proteinuria, glycosuria) include some fatal failures. Cases of acute renal failure have been reported after only one or two doses of Vistide®. In accordance with the invention, cidofovir is used for the treatment and/or prevention and/or palliation of neoplasms. The invention enables using lower doses than those currently approved for the treatment of CMV retinitis. It is believed that the doses of cidofovir needed to implement the invention are so low that most of the above-mentioned side effects, and at least the renal toxicity side effects, will be avoided.

1.2. Acycloguanosine (e.g.. Aciclovir®):

Acycloguanosine is 2-amino-9-((2-hydroxyethoxy)methyl)-1H-purin-6(9H)-one with the molecular formula of $C_8H_{11}N_5O_3$ and a MW of 225.21 g/mol. It is an acyclic nucleoside analogue (i. e., a guanosine analogue wherein the sugar ring has been replaced by an open-end carbohydrate structure).

When administered to an animal, more particularly to a mammal, still more particularly to a human, cycloguanosine is selectively converted into a monophosphate form (acycloguanosine monophosphate or acyclo-GMP) by viral thymidine kinase. Subsequently, the monophosphate form is further phosphorylated into the active triphosphate form, acycloguanosine triphosphate (acyclo-GTP), by cellular kinases. Acyclo-GTP is a very portent inhibitor of viral DNA polymerase; it has approximately 100 times greater affinity for viral than cellular polymerase. As a substrate, acyclo-GTP is incorporated into viral DNA, resulting in chain termination.

Cycloguanosine is active against most species of the herpesvirus family, more particularly Herpes Simplex Virus type I (HSV-1), Herpes Simplex Virus type II (HSV-2), Varicella Zoster Virus (VZV), EBV, and, but to a lesser extent, CMV.

Acycloguanosine is currently marketed under various trade names such Aciclovir® (Sanofi-Aventis).

Acycloguanosine is commonly marketed as tablets: (200 mg, 400 mg, 800 mg and 1 gram), topical cream (5%), intravenous injection (25 mg/mL) and ophthalmic ointment (3%).

For the treatment of herpes virus infection, the approved doses of acycloguanosine are of 200 mg to 800 mg 5 times daily for 5 to 10 days (adult doses).

Common adverse drug reactions associated with systemic acycloguanosine therapy (oral or intravenously) include: nausea, vomiting, diarrhea and/or headache. In high doses, hallucinations have been reported. Additional common adverse effects, when acycloguanosine is administered intraveneously, include encephalopathy, renal impairment and injection site reactions.

In accordance with the application, acycloguanosine is used for the treatment and/or prevention and/or palliation of neoplasms. Preferred forms of acycloguanosine are those which are suitable for oral and/or intraveneous administration.

The application enables using lower doses than those currently approved for the treatment of herpes virus infection. It is believed that the doses of Acycloguanosine needed to implement the application are so low that most of the above-mentioned side effects, and at least the renal toxicity side effects, will be avoided.

1.3. Foscarnet (*e.g.,* Foscarvir®):

Foscarnet is phosphonoformic acid. It is commercially available as a trisodium hexahydrate salt, e.g., under the trademark Foscarvir® (intravenous administration). Its chemical formula is $Na_3CO_5P.6H_2O$ and has a MW of 300.1. It is a pyrophosphate analogue.

Foscarnet is indicated for induction and maintenance therapy of CMV retinitis in patients with AIDS. It is also indicated for the treatment of acycloguanosine-resistant mucocutaneous HSV infections in immunocompromised patients.

Foscarnet is not recommended for treatment of CMV infections other than retinitis or for use in non-AIDS or non-immunocompromised patients.

For the clinically approved application, *i. e.,* the palliation of CMV retinitis in patients with AIDS, induction doses of 180 mg/kg every 8 hours for 24 hours, followed by maintenance treatment with 60 to 90-120 mg/kg/day for 3 weeks are recommended.

The main adverse effect of foscarnet is its renal toxicity.

## 2. EGFR inhibitor(s):

The term "EGFR" is herein intended in accordance with its ordinary meaning in the field, *i:e., as* meaning Epidermal Growth Factor Receptor or ErbB-1 (HER1 in humans). It is a member of the ErbB family of receptors. Naturally-occuring EGFR is expressed on the cell surface and is activated by binding of specific ligands, including Epidermal Growth Factor (EGF) and Transforming Growth Factor $\alpha$ (TGF$\alpha$). Upon activation by its growth factor ligand(s), EGFR undergoes a transition from an inactive monomeric form to an active homodimer. EGFR may also pair with other member(s) of the ErbB receptor family, such as ErbB2/Her2/neu, to create an activated heterodimer, and/or form clusters of activated EGFR forms.

EGFR overexpression and/or overactivity have been associated with a number of cancers, including lung cancer, anal cancers and glioblastoma multiforme.

The identification of EGFR as an oncogene has led to the development of anticancer therapeutics directed against EGFR, including gefitinib *(e.g.,* Iressa®) and erlotinib (*e.g.*, Tarceva®) for lung cancer, and Cetuximab (e.g., Erbitux®) for colon cancer.

The term "EGFR inhibitor" is herein intended in accordance with its ordinary meaning in the field. More particularly, an EGFR inhibitor is herein intended as a product (*e.g.,* a compound, a molecule, a complex, a composition), which inhibits signal transduction through EGFR.

Illustrative EGFR inhibitors comprise EGFR antagonists, i.e., products that bind to EGFR on its intracellular domain and/or on its transmembranar domain and/or on its intracellular domain (e.g., on its kinase domain), such that signal transduction through EGFR is inhibited, preferably competitively inhibited.

Such an antagonist may e.g., an antibody, a monoclonal antibody, an antibody fragment (such as a F(ab')2, Fab, Fv or CDR fragment), or a structure derivable from an antibody by re-engineering (such as a scFv, a Heavy Chain antibody -or HCAb-, a VHH or a humanized antibody).

Alternatively, such an antagonist may *e.g.*, be a quinazoline compound.

In an embodiment of the application or invention, said EGFR antagonist is a ligand, which binds to the extracellular domain of EGFR and, which inhibits, preferably competitively inhibits, the binding of EGF and/or TGF-alpha, preferably of EGF and TGF-alpha, on EGFR. Illustrative of such EGFR antagonists are anti-EGFR antibodies, more particularly anti=EGFR monoclonal antibodies (mAbs), such as anti-EGFR mAbs that target EGFR extracellular domain, or fragments of such antibodies (such as the F(ab')2, Fab, Fv or CDR fragments thereof), or structures derivable from such antibodies by re-engineering (such as scFv, Heavy Chain antibodies, VHH and humanized antibodies). Examples of such EGFR antagonists comprise the mAbs cetuximab (Erbitux®) and panitumumab (Vectibix®).

In an embodiment of the application or invention, said EGFR antagonist is a product, which binds to the intracellular domain of EGFR, more particularly to an EGFR kinase domain, still more particularly to an EGFR tyrosine kinase domain and, which inhibits, preferably competitively inhibits, the EGF-mediated activation of EGFR. The induction of this inhibition is a means to disrupt one or several of the pro-tumor processes which are regulated by the EGFR-signaling pathways in tumor cells and/or in stromal cells in the tumor microenvironment. Examples of such EGFR antagonists comprise erlotinib (Tarceva®) and gefitinib (*e.g.*, Iressa®).

Other illustrative EGFR inhibitors comprise antagonists of one or several EGFR downstream effectors), *i.e.*, products that bind to one or several EGFR downstream effector(s), such that signal transduction through EGFR is inhibited, preferably competitively inhibited.

In addition to the structural unit(s) that inhibits signal transduction through EGFR, an EGFR inhibitor may comprise other structural units, such as units that improves its cell or molecular specificity, e.g., to more specifically target those cells that are tumor cells.

In an embodiment of the application or invention, said at least one EGFR inhibitor is cetuximab (Erbitux®), or panitumumab (Vectibix®), or erlotinib (Tarceva®), or gefitinib (*e.g.*, Iressa®), or a salt thereof, advantageously cetuximab (Erbitux®) or erlotinib (Tarceva®), or a salt thereof, more particularly cetuximab (Erbitux®), or a salt thereof.

## 2.1. Cetuximab (Erbitux®):

Cetuximab (also sometimes referred to as C225) is a recombinant, human/mouse chimeric monoclonal antibody that binds specifically to the extracellular domain of the human EGFR. It is an EGFR antagonist composed of the Fv regions of a murine anti-EGFR antibody with human IgGl heavy and kappa light chain constant regions. Cetuximab is produced in mammalian (murine myeloma) cell culture. It has a MW of 152 kDa. It is described in US 6,217,866 B1. (ATCC. 9763; ATCC 9764) and its non-US counter-part patent applications or patents.

Cetuximab is commercialized under the trademark Erbitux®. It is manufactured by ImClone Systems Inc. (Branchburg, NJ 08876; U.S.A.) and distributed and marketed by Bristol-Myers Squibb Company (Princeton, NJ 08543;

U.S.A.).

Erbitux® is commercialized as an injectable liquid containing cetuximab particulates. Erbitux® is supplied at a concentration of 2 mg/mL single-use vials.

Erbitux® is currently indicated in the treatment of Squamous Cell Carcinoma of the Head and Neck (SCCHN) and of colorectal carcinomas as follows:

- in combination with radiation therapy, for treatment of locally or regionally advanced SCCHN;
- for treatment of recurrent or metastatic SCCHN progressing after platinum-based therapy;
- as a single agent, for treatment of EGFR-expressing metastatic colorectal carcinoma after failure of both irinotecan- and oxaliplatin-based regimes or in patients who are intolerant to irinotecan-based regimens;
- in combination with irinotecan, for treatment of EGFR-expressing metastatic colorectal carcinoma in patients who are refractory to irinotecan-based chemotherapy.

For said treatments of SCCNH and for said treatments of colorectal carcinoma, the recommended daily dose of Erbitux® is 400 mg/m$^2$ as the initial dose, and 250 mg/m$^2$ for the subsequent weekly doses.

Cetuximab binds specifically to the EGFR both on normal and cancer cells, and competitively inhibits the binding of EGF and other ligands such as transforming growth factor-alpha.

The binding of cetuximab to the EGFR blocks phosphorylation and activation of receptor-associated kinases, resulting in inhibition of cell growth, induction of apoptosis and decreased matrix metalloproteinase and vascular endothelial growth factor production. However, at the doses it is used for said treatments of SCCHN (in combination with radiation or platinum-based therapy), as well as at the doses it is used for said treatments of metastatic colorectal carcinoma (in monotherapy or in combination with irinotecan-based therapy), cetuximab is associated with various adverse reactions, including serious adverse reactions such as:

- skin reactions such as rash, hypomagnesaemia, dermatologic toxicity and radiation dermatitis,
- fever, chills, dizziness, difficulty in breathing,
- cardiopulmonary arrest;
- sepsis;
- renal failure;
- interstitial lung disease; and
- pulmonary embolus.

Approximately 90% of severe infusion reactions occurs with the first infusion despite premedication with antihistamines.

Skin reactions are seen in more than 80% of the patients. According to a European study (Cunningham *et al.* 2004), the rate of response to the combined treatment with Erbitux® and irinotecan is independent of the cell EGFR, expression level.

### 2.2. Panitumumab (Vectibix®):

Panitumumab is a recombinant, fully human IgG2 kappa monoclonal antibody that binds specifically to the human epidermal growth factor receptor (EGFR). It is commercialized under the trademark Vectibix®, and is manufactured by Amgen Inc. (U.S.A.). It is commercialized as an injectable solution for intravenous use.

Vectibix® is indicated as a single agent for the treatment of EGFR-expressing, metastatic colorectal carcinoma with disease progression on or following fluoropyrimidine-, oxaliplatin-, and irinotecan-containing chemotherapy regimens. Vectibix® is covered by US patent 6,235,883, the US child application(s) and patent(s) thereof, as well as by the non-US counterpart patents and patent applications thereof. Currently-available clinical data report severe dermatologic toxicity and infusion reactions. Dermatologic toxicities occurred in 89% of patients and were severe (NCI-CTC grade 3 and higher) in 12% of patients receiving Vectibix® monotherapy. Withhold or permanent discontinuation of Vectibix® is recommended for dermatologic toxicities that are grade 3 or higher or are considered intolerable. The clinical manifestations included, but were not limited to, dermatitis acneiform, pruritus, erythema, rash, skin exfoliation, paronychia, dry skin, and skin fissures. Subsequent to the development of severe dermatologic toxicities, infectious complications, including sepsis, septic death, and abscesses requiring incisions and drainage were reported. Severe infusion reactions included anaphylactic reactions, bronchospasm, and hypotension.

Vectibix® is currently not indicated for use in combination with chemotherapy. In an interim analysis of a randomized (1:1) clinical trial of patients with previously untreated metastatic colorectal cancer, the addition of Vectibix® to the combination of bevacizumab and chemotherapy resulted in decreased overall survival and increased incidence of NCI-CTC grade 3-5 (87% vs. 72%) adverse reactions. In a single-arm study of 19 patients receiving Vectibix® in combination with IFL protocol (5-fluorouracile 500 mg/m$^2$, leucovorine 20 mg/m$^2$ and irinotecan 125 mg/m$^2$), the incidence of NCI-CTC grade 3-4 diarrhea was 58%; in addition, grade 5 diarrhea occurred in 1 patient.

Permanent discontinuation of Vectibix® therapy is recommended for patients developing interstitial lung disease, pneumonitis, or lung infiltrates.

### 2.3. Erlotinib (Tarceva®):

Erlotinib is a quinazolinamine with the chemical name N-(3-ethynylphenyl)-6,7-bis(2-methoxyethoxy)-4-quinazolinamine. It is a kinase inhibitor indicated for the treatment of;

- locally advanced or metastatic non-small cell lung cancer (NSCLC) after failure of at least one prior chemotherapy regimen;
- first-line treatment of patients with locally advanced or metastatic pancreatic cancer, in combination with gemcitabine.

It is commercialized under the trademark Tarceva® wherein it is contained in its hydrochloride salt form.

Tarceva® is manufactured by Schwartz Pharma Manufacturing (Seymour, IN 47274; U.S.A.) for OSI Pharmaceuticals, Inc. (Melville, NY 11747; U.S.A.), and distributed by Genentech USA, Inc. (San Fransisco, CA 94080-4990; U.S.A.).

Tarceva® is commercialized in tablets in three dosage strengths (25mg, 100mg or 150 mg erlotinib).

The recommended daily dose of Tarceva® is 150 mg for said treatment of non-small cell lung cancers, and is 100 mg for said treatment of pancreatic cancers.

The mechanism of clinical anti-tumor action of erlotinib is not fully characterized. Erlotinib inhibits the intracellular phosphorylation of tyrosine kinase associated with EGFR.

However, at the doses it is used for said treatment of non-small cell lung cancers or of pancreatic cancers, erlotinib is associated with various adverse reactions, including serious adverse reactions such as severe diarrhea (unresponsive to loperamide), severe skin reactions, and serious Interstitial Lung Disease (ILD)-like events, including fatalities.

### 2.4. Gefitinib (*e.g.*, Iressa®):

Gefitinib is 4-Quinazolinamine, N-(3-chloro-4-fluorophenyl)-7-methoxy-6-[3-4-morpholin]propoxy]. Its chemical formula is $C_{22}H_{24}ClFN_4O_3$ with a MW of 446.9.

It is commercialized under the trademark Iressa®, wherein it is contained at 250 mg per tablet.

The mechanisms of action of gefitinib are not fully characterized. It inhibits the intracellular phosphorylation of tyrosine kinases associated with EGFR,

Clinical studies suggest that gefitinib could be useful as third line therapy (250 mg or 500 mg daily dose) in Non-Small Cell Lung Cancer (NSCLC) patients with disease progression on platinum and docetaxel therapies or who had had unacceptable toxicity on these agents.

Gefitinib has a pulmonary toxicity and a renal toxicity.

### 3. Administration features:

The formulation of a combination or combined preparation of the application or invention is dependent upon the administration route, in accordance with the usual practice in the field.

In a combination or combined preparation of the application or invention, said at least one antiviral agent and said at least one EGFR inhibitor can e.g., be formulated in a form suitable for parenteral, transmucosal, enteral or topical administration, advantageously for parenteral or transmucosal administration.

Said at least one antiviral agent and said at least one EGFR inhibitor may be formulated for different administration routes (for example, in a form suitable for parenteral administration for said at least one antiviral agent, and in a form suitable for transmucosal administration for said at least one EGFR inhibitor). Alternatively, they can be both formulated for the same administration route (for example, both in a form suitable for parenteral administration).

A preferred parenteral administration is the intravenous or intrathecal administration.

In an embodiment of the application or invention, a combination or combined preparation of the application or invention is formulated in a form suitable for, or is intended for systemic or intratumoral administration.

The determination of the dose regimen of said at least one antiviral agent and said at least one EGFR inhibitor is determined by the person of ordinary skill in the art so as to achieve the desired effect, notably a decrease in the survival of the neoplastic cells.

The dose regimen, more particularly the dose amount (or dose per administration) of said at least one antiviral agent and said at least one EGFR inhibitor, is advantageously such that said at least one antiviral agent and said at least one EGFR inhibitor exert a synergistic effect, more particularly a synergistic decrease or inhibition of the survival of neoplastic cells.

For example, at a fixed dose amount of said at least one EGFR inhibitor, the dose amount of said at least one antiviral agent can be increased up to the point where said synergistic effect is observed and/or the frequency of administration of each dose of said at least one antiviral agent can be increased up to said synergistic point.

For example, the dose regimen, more particularly the dose amount of said at least one antiviral agent and said at least one EGFR inhibitor is such that said at least one antiviral agent reveals or stimulates the EGFR inhibition induced by the EGFR inhibitor.

For example, the dose regimen, more particularly the dose amount of said at least one antiviral agent and said at least one EGFR inhibitor is such that said at least one antiviral agent increases the sensitivity of the neoplastic cells to the EGFR inhibition induced by the EGFR inhibitor.

Objective solid tumor response can be measured or monitored *e.g.,* using the Response Evaluation Criteria in Solid Tumors (RECIST); *cf.* Therrasse *et al.* 2000, and Eisenhauer *et al.* 2009.

The dose regimen of said at least one antiviral agent and/or of said at least one EGFR inhibitor (dose amount at each administration and/or frequency of administration of each dose) should also be adjusted so as to limit the induced toxicity to a level that is acceptable for the health of the subject(s).

Generally speaking, the administration of said at least one antiviral agent and/or of said at least one EGFR inhibitor should be discontinued or the dose of said at least one antiviral agent should be reduced as soon as a Serious Adverse Event (SAE) is observed. A SAE is any untoward medical occurrence that at any dose is life-threatening, or requires patient hospitalization or prolongation of existing hospitalization, or results in permanent disability or serious temporary incapacity, or results in a congenital abnormality, a birth defect or abortion, or is medically significant.

More particularly, the administration of said at least one antiviral agent and/or of said at least one EGFR inhibitor should be discontinued or the dose of said at least one antiviral agent should be reduced as soon as grade 3 or 4 toxicity or any recurrent or persistent grade 2 toxicity is observed.

Toxicity is herein meant as an adverse event as defined and as graded in accordance with the National Cancer Institute Common Terminology Criteria for Adverse Events (CTCAE), version 3.0, published 9 August 2006 (http://ctep.cancer.gov/reporting/ctc.html).

Among the various toxicities, which can be induced by antiviral agents such as cidofovir, renal and urinary disorders, *e.g.*, increase in urine protein level (proteinuria) and increase of blood creatine (creatinemia), should be cautiously monitored.

Preferably, the patient receives an antihistamine treatment as a prophylactic treatment before administration of an EGFR inhibitor such as the mAb cetuximab (Erbitux®), so as to at least limit anaphylactic reactions. Appropriate antihistamine treatments notably comprise diphenhydramine (*e.g.*, Benadryl® or Dimedrol®), for example a 25-50 mg intravenous injection of diphenhydramine 30 min before each administration of an EGFR inhibitor such as the mAb cetuximab (Erbitux®).

The particular dose amount to be administered.is also dependent on the particular antiviral agent and the particular EGFR inhibitor being used, and of the particular condition and reactions of the subject(s) to be treated.

The means of the application or invention enable to reveal or stimulate the EGFR inhibition induced by said at least one EGFR inhibitor. More particularly, they enable to overcome the resistance(s) to EGFR blockage that are frequently observed when an EGFR inhibitor (such as cetuximab, e.g., Erbitux®) is administered to the subject(s). Hence, in a particular embodiment of the application or invention, said at least one EGFR inhibitor is administered in a dose amount that is lower than currently used in the prior art, more particularly lower than the currently approved dose for said EGFR inhibitor.

For example, when said at least one EGFR inhibitor is cetuximab (Erbitux®) or panitumumab (e.g., Vectibix®), a dose amount leading to a maximum plasmatic concentration (e.g., one hour after administration of the dose) of less than 100 μg/mL, advantageously of less than 40 μg/mL, for example 10 to 40 μg/mL can be used in accordance with the application or invention.

For example, when said at least one EGFR inhibitor is erlotinib (*e.g.*, Tarceva®) or gefitinib (*e.g.*, Iressa®), a dose amount leading to a maximum plasmatic concentration *(e.g.,* one hour after administration of the dose) of less than 3 nM, advantageously of 0.1-2.5 nM, can be used.

As previously described and above illustrated, the means of application or invention further enable to obtain a particularly unexpected effect when said neoplastic cells are virus-positive, more particularly HPV-positive: a much lower amount of antiviral agent than expected is required to obtain said synergistic effect.

In an embodiment of the application or invention, said at least one antiviral agent is administered at a dose leading to a maximum plasmatic concentration (*e.g.*, 1 hour after administration of the dose) of 10 μg/mL or less, for example of 1-10 μg/mL, advantageously of 5.5 μg/mL or less, for example of 1-5.5 μg/mL, more particularly of 5 μg/mL or less, for example of 1-5 μg/mL. Such doses are more particularly suitable when said at least one antiviral agent is cidofovir (*e.g.*, Vistide®).

In a human body, a dose of less than 3 mg/kg, for example of 0.5-2.5 mg/kg is more particularly contemplated.

When said at least one antiviral agent is foscarnet (*e.g.*, Foscarvir®), a dose amount leading to a maximum plasmatic concentration (*e.g.*, 1 hour after administration of the dose) as low as 50-800 nM can be contemplated.

When said at least one antiviral agent is or comprises cidofovir, said EGFR inhibitor preferably is or comprises cetuximab.

When said at least one antiviral agent is or comprises foscarnet, said EGFR inhibitor preferably is or comprises erlotinib.

The frequency of administration of each dose of said at least one antiviral agent and said at least one EGFR inhibitor is dependent on the particular antiviral agent and the particular EGFR inhibitor being used. It is also dependent of the subject(s) for which said dose regimen is intended. The sequence of administration of one product relative to the other product can be determined and adjusted depending on the particular condition and reactions of the subject(s) to be treated.

In an embodiment of the application or invention, said at least one EGFR inhibitor is administered at a higher frequency than the antiviral agent.

For example, said at least one EGFR inhibitor is administered once per week, and said at least one antiviral agent once every two weeks. This is more particularly the case when said at least one EGFR inhibitor is or comprises cetuximab and said at least one antiviral agent is or comprises cidofovir.

An illustrative scheme of administration frequency, which is particularly suitable for cidofovir as antiviral agent and cetuximab as EGFR inhibitor, is a cycle comprising a week "n" where only the EGFR inhibitor is administered and a week "n+1" where both the antiviral agent and the EGFR inhibitor are administered, for example as follows:

- EGFR inhibitor but no antiviral agent on week 1;
- antiviral agent and EGFR inhibitor on week 2;
- EGFR inhibitor but no antiviral agent on week 3;
- antiviral agent and EGFR inhibitor on week 4;
- etc. until a satisfactory tumor response and/or said synergistic effect is obtained.

In a particular embodiment of the application or invention, when said at least one antiviral agent (more particularly cidofovir) and said at least one EGFR inhibitor (more particularly cetuximab) are administered during the same week, more particularly on the same day, the antiviral agent is administered first, before said EGFR inhibitor is administered.

### 4. Groups of neoplasms; groups of subjects:

The combination of the application can be used in the treatment and/or prevention and/or palliation of neoplasms, which are:

- virus-positive (*i.e.*, said neoplasms comprise cells containing virus(es)), and/or
- virus-associated (*i.e.*, said neoplasms do not necessarily comprise cells containing any virus -or at least not in a substantial or detectable amount-, but the presence or development of said neoplasm in said organism is at least partially due to the (present, recent or past) presence or development of said virus(es) in said organism).

The combination of the application or invention can be used in the treatment and/or prevention and/or palliation of neoplasms, which are virus-negative and not virus-associated.

In an aspect of the application, the combination of the application or invention is used in the treatment and/or prevention and/or palliation of neoplasms, which are virus-positive or virus-negative, more particularly *Papillomaviridae-positive* or *Papillomaviridae-negative,* still more particularly HPV-positive or HPV-negative, advantageously HPV-positive.

In an embodiment of the application, the combination of the application or invention is used in the treatment and/or prevention and/or palliation of neoplasms, which are, and wherein the organism(s) carrying said neoplasms is(are), *Papillomaviridae*-negative and *Herpesviridae-negative,* more particularly virus-negative, *e.g.,* for the past 2 months before said neoplasms receive the combination of the application or invention. In another embodiment of the application or invention, the combination of the application or invention is used in the treatment and/or prevention and/or palliation of neoplasms, which are, and/or wherein the organism(s) carrying said neoplasms is(are), *Papillomaviridae*-positive and/or *Herpesviridae*-positive, more particularly:

- *Papillomaviridae*-positive and *Herpesviridae*-positive, or *Papillomaviridae*-positive and *Herpesviridae*-positive; or
- *Papillomaviridae-negative* and *Herpesviridae*-positive;

*e.g.,* shortly before *(e.g.,* less than 2 months before), or at the time when said neoplasm receives the combination of the applications or invention.

In a particular embodiment of the application, the combination of the application or invention is used in the treatment and/or prevention and/or palliation of neoplasms, which are *Papillomaviridae*-positive and/or *Herpesviridae*-positive, more particularly *Papillomaviridae*-positive.

According to an advantageous embodiment of the application, said *Papillomaviridae* virus(es) is(are) (at least or specifically) High Risk (HR) HPV, more particularly (at least or specifically) HPV16 and/or HPV18 and/or HPV31 and/or HPV45, still more particularly (at least or specifically) HPV16 and/or HPV18.

According to an advantageous embodiment of the application, said *Herpesviridae* virus is (at least or specifically) EBV.

Complementarily or alternatively to said features of virus status, said neoplasms and the organism(s) carrying them can be EGFR-positive; or said neoplasms and/or the organism(s) carrying them can be EGFR-negative. In accordance with a particular embodiment of the application, said neoplasms and/or the organism(s) carrying them are EGFR-positive.

Said neoplasms can be solid neoplasms or liquid neoplasms. In accordance with a particular embodiment of the application, said neoplasms are solid neoplasms.

In a particular embodiment of the application, said neoplasms are or comprise Squamous Cell Carcinoma of the Head and Neck (SCCHN) including recurrent and/or metastatic SCCHN, cervix tumor(s), vulvar tumor(s), penile tumor(s), anal tumor(s), colorectal tumor(s), lung tumor(s), such as non-small cell lung cancer (NSCLC), more particularly locally advanced or metastatic NSCLC, and nasopharyngeal tumor(s).

In a more particular embodiment of the application or invention, said neoplasms are any group of two specific neoplasms, more particularly any one single specific neoplasm, selected from this particular list of neoplasms, advantageously recurrent and/or metastatic. SCCHN, and/or locally advanced or metastatic NSCLC.

The neoplasms listed in said particular list or selected from said particular list can have any of the above-described features.

In a particular embodiment of the application, said SCCHN, recurrent and/or metastatic SCCHN, cervix tumor(s), vulvar tumor(s), penile tumor(s), anal tumor(s), colorectal tumor(s), lung tumor(s), NSCLC, and locally advanced or metastatic N-SCLC, are *Papillomaviridae*-positive, advantageously HPV-positive, optionally EGFR-positive, as above described.

In a particular embodiment of the application, said nasopharyngeal tumor(s) are *Herpesviridae*-positive (advantageously EBV-positive), optionally EGFR-positive, as above described.

The combination of the application is intended:

- for simultaneous, separate or sequential use in a medical treatment and/or prevention and/or palliation of neoplasms in subjects in need thereof, and/or
- for simultaneous, separate or sequential administration to subjects in need thereof.

In an embodiment of the application or invention, said subjects are animals, still more particularly mammals, advantageously humans.

In an aspect of the application, said subjects are specific group(s) of subjects, more particularly of animals, still more particularly of mammals, advantageously of humans.

In an embodiment of the application, said specific group is the group of those subjects, who are non-responsive, or have become resistant to:

- the administration of an EGFR inhibitor (*e.g.*, an EGFR inhibitor as above-defined, more particularly cetuximab), and/or to
- a systemic chemotherapy,

more particularly to a systemic chemotherapy.

In an embodiment of the application, said administration of EGFR inhibitor is a monotherapy, or is a bi-therapy combined with the administration of a systemic chemotherapy. In a particular embodiment of the application or invention, said EGFR inhibitor administration is a monotherapy.

In an embodiment of the application, said systemic chemotherapy is a monotherapy, or is a bi-therapy combined with the administration of an EGFR inhibitor (*e.g.,* an EGFR inhibitor as above-defined, more particularly cetuximab). In a particular embodiment of the application or invention, said systemic chemotherapy is a monotherapy.

In an embodiment of the application, said systemic chemotherapy comprises or is the administration of a taxane (or of a taxane-based or taxane-comprising chemotherapy drug), or the administration of a platinum derivative (or of chemotherapy drug based on, or comprising, a platinum derivative). Said taxane advantageously is paclitaxel or docetaxel, more particularly paclitaxel. Said platinum derivative advantageously is cisplatin.

In a particular embodiment of the application, said chemotherapy is the intravenous administration of 50 to 100 mg/m$^2$ of cisplatin in one single injection, once every four weeks for nine months.

A subject is considered to be non-responsive or to have become resistant to said treatment(s), when he/she does not show a therapeutic improvement either in the course of said treatment(s) or in the month following the end of said treatment(s), more particularly in the course of said treatment(s). Said therapeutic improvement preferably is the inhibition of the proliferation of the neoplastic cells and/or the decrease in the survival of the neoplastic cells.

In an embodiment of the applications, said specific group is the group of those subjects, for whom a systemic chemotherapy is contraindicated.

In a particular embodiment of the application, said specific group is the group of those subjects, for whom the administration of a taxane (or of a taxane-based or taxanecomprising chemotherapy drug), and/or the administration of a platinum derivative (or of chemotherapy drug based on, or comprising, a platinum derivative) is contraindicated.

In a more particular embodiment of the application, said specific group is the group of those subjects, for whom the administration of a platinum derivative (or of chemotherapy drug based on, or comprising, a platinum derivative) is contraindicated.

Said taxane advantageously is paclitaxel or docetaxel, more particularly paclitaxel. Said platinum derivative advantageously is cisplatin.

Said group of subjects, for whom a systemic chemotherapy is contraindicated, more particularly comprises subjects:

- who are hypersensitive to platinum and/or to a platinum derivative, more particularly to cisplatin; and/or
- who are hypersensitive to taxane, more particularly to paclitaxel and/or docetaxel; and/or
- who are pregnant; and/or
- who have a renal insufficiency; and/or
- who have an auditory insufficiency; and/or
- who receive or have to receive a nephrotoxic and/or an ototoxic drug; and/or
- who receive or have to receive an attenuated virus, such as the yellow fever vaccine.

In an embodiment of the application, said specific group is the group formed by:

- those subjects, who are non-responsive, or have become resistant to systemic chemotherapy and/or to the administration of an EGFR inhibitor, as above defined, and by
- those subjects, for whom a systemic chemotherapy is contraindicated, as above-defined.

In the application, the term "comprising", which is synonymous with "including" or "containing", is open-ended, and does not exclude additional, unrecited element(s), ingredient(s) or method step(s), whereas the term "consisting of" is a closed term, which excludes any additional element, step, or ingredient which is not explicitly recited. The term "essentially consisting of" is a partially open term, which does not exclude additional, unrecited element(s), step(s), or ingredient(s), as long as these additional element(s), step(s) or ingredient(s) do not materially affect the basic and novel properties of the means of the application.

The term "comprising" (or "comprise(s)") hence includes the term "consisting of" ("consist(s) of"), as well as the term "essentially consisting of" ("essentially consist(s) of"). Accordingly, the term "comprising" (or "comprise(s)") is, in the application, meant as more particularly encompassing the term "consisting of" ("consist(s) of"), and the term "essentially consisting of" ("essentially consist(s) of").

In an attempt to help the reader of the present application, the description has been separated in various paragraphs or sections. These separations should not be considered as disconnecting the substance of a paragraph or section from the substance of another paragraph or section. To the contrary, the present description encompasses all the combinations of the various section, paragraphs and sentences that can be contemplated.

The following examples are offered by way of illustration, and not by way of limitation.

## EXAMPLES

## EXAMPLE 1: IN VITRO ASSAYS

**Material and Methods**

Cell cultures:

The cell lines are available from the American Type Culture Collection (ATCC):

- HeLa cells (human HPV-positive cervical carcinoma cells): ATCC n°CCL-2;
- Me 180 cells (human, HPV-positive cervical carcinoma cells): ATCC n°HTB-33;
- C33A cells (human HPV-negative cervical carcinoma cells): ATCC n°HTB-31.
- H460 cells (human HPV-negative lung carcinoma cells): ATCC n°HTB-177.

HeLa cells were grown in the GIBCO® Dulbecco's Modified Eagle Medium containing 0.11 g/L sodium pyruvate and 4.5 g/L glucose (GIBCO® DMEM available from Invitrogen under catalog reference number 31966021), which had been supplemented with 10% Fetal Calf Serum (FCS), 1% penicillin/streptomycin and 1% glutamine.

The HeLa cells contained 10-50 copies of integrated HPV18.

Mel80 cells were grown in the GIBCO® McCoy's 5A medium (available from Invitrogen under catalog reference number 26600023), supplemented with 10% FCS, 1% penicillin/streptomycin and 1% glutamine.

C33A cells were grown in the GIBCO® Modified Eagle Medium (GIBCO® MEM available from Invitrogen under catalog reference number 21090022), which had been supplemented with 10% FCS, 1% penicillin/streptomycin and 1% glutamine.

H460 cells were grown in the GIBCO® RPMI_1640 (GIBCO® RPMI-1640 available from Invitrogen under catalog reference number), which had been supplemented with 10% FCS, 1% penicillin/streptomycin and 1% glutamine.

FCS is available from Invitrogen under catalog reference number 10270088.

GIBCO® penicillin/streptomycin is available from Invitrogen under catalog reference number 15140-122.

Glutamine is available from Invitrogen under catalog reference number 25030024.

The cell lines were grown in humid incubators at 37°C under 5%.$CO_2$.

Drugs

*EGFR inhibitors:*

Cetuximab (Erbitux®) is available from Merck Germany (solution at 5mg/mL).

Erlotinib (Tarceva®) at 150 mg per tablet is available from Roche, which was dissolved in DiMethylSulfOxide (DMSO) to obtain a stock solution at 50 mM.

*Antiviral agents:*

Cidofovir (Vistide®) is available from Pharmacia Upjohn (Guyancourt les Yvelines; France) as a 75 mg/mL solution. Vistide® had been diluted in de-ionized water to obtain a 1 mg/mL cidofovir stock solution.

Foscarnet sodium (Foscarvir®) is available from AstraZeneca as a 6g/250mL solution.

Clonogenicity tests:

The change in the clonogenicity of the cell lines (HeLa, Me180, C33A and H460) was determined on cultures in 6-well plates of 200 to 1,000 cells/well receiving one of the following treatments:

- cidofovir alone added to medium (one dose once per day; 1, 5 or 10 µg/mL);
- cetuximab alone added to medium (one dose once per day; 10 or 50 µg/mL);
- cidofovir and cetuximab added to medium (combinations of the individual doses, once per day);
- erlotinib alone added to medium (one dose once per day; 0.1, 0.5 µM/mL);
- foscarnet sodium alone added to medium (one dose once per day; 200 µM/mL);
- foscarnet sodium and erlotinib added to medium (combinations of the individual doses, once per day);
- cidofovir and erlotinib (combinations of the individual doses, once per day);
- foscarnet sodium and cetuximab (combinations of the individual doses, once per day).

Each of said treatments last the 10-15 days of the clonogenic assay.

The culture medium has not been replaced during the assay.

Control = without addition of antiviral agent (cidofovir, foscarnet sodium) and without addition of EGFR inhibitor (cetuximab, erlotinib).

The cultures were stopped by fixing and staining with crystal violet (methanol 80%, formaldehyde 10%, crystal violet 1.25 g), when the clones reached 50 cells. The clones were then counted and related to the number of cells cultured to obtain the fraction of surviving cells.

Bliss additivity model:

The Bliss additivity model was used to classify the effect of the drug combination us synergistic, additive or antagonist.

A theoretical curve of the expected effect of the combination was calculated from the equation:

$$\text{Bliss effect} = \text{effect A} + \text{effect B} - \text{effect A} \times \text{effect B,}$$

where effect A and effect B are the effects of drugs A and B alone at specific concentrations.

Here, the Bliss effect is the expected effect when the combination of the two drugs is exactly additive. If the observed effect (the experimentally measured effect of the combination) is lower than the Bliss effect, the effect of the combination is said to be antagonist (delta = observed effect - Bliss effect is a negative value). If the observed effect is greater than the Bliss effect, the effect of the combination is synergistic (delta is positive).

For the dose-effect curves, delta was calculated for increasing doses of drug A (e.g., cidofovir) combined with a constant dose of drug B (e.g., cetuximab). The data are expressed as a percentage decrease in cell viability beyond that which would be expected if the association were strictly additive.

## RESULTS

### Effect of a cidofovir+cetuximab combination on tumour growth of HPV-positive and HPV-negative neoplastic cells *in vitro*

**Clonogenicity tests:**

A combination of an antiviral agent (cidofovir) with an inhibitor of EGFR (cetuximab) was investigated to ascertain whether it had an effect on cell mortality. To this end, in vitro clonogenicity tests were carried out on human HPV-positive cervical carcinoma cells cells (HeLa cells; Me180 cells).

Figures 1A, 1B and 1C illustrate the results obtained with HeLa cells.

Cidofovir alone had an effect on HeLa cell clonogenicity when used at a concentration of at least 5 $\mu$g/mL.

At the concentration of 5 $\mu$g/mL, HeLa cell survival reduced by about 15% (*cf.* Figure 1A). When the concentration of cidofovir reached 10 $\mu$g/mL, the clonogenicity had reduced by 25% compared to the control (*cf.* Figure 1A).

Cetuximab alone at 10 $\mu$g/mL had no effect on HeLa cell clonogenicity (*cf.* Figure 1B). When the antiviral agent was combined to the EGFR antagonist, there was a much greater reduction in HeLa cell clonogenicity, even at low doses of cidofovir.

Cidofovir at 1 $\mu$g/mL with cetuximab at 10 $\mu$g/mL resulted in a reduction in HeLa cell clonogenicity of about 50% compared to the control (*cf.* Figure 1C).

Cidofovir at a dose of 5 $\mu$g/mL with cetuximab at 10 $\mu$g/mL resulted in a reduction in HeLa cell clonogenicity of about 55% compared to the control, respectively (*cf.* Figure 1C).

Cidofovir at a dose of 10 $\mu$g/mL with cetuximab at 10 $\mu$g/mL resulted in a reduction in HeLa cell clonogenicity of about 60% compared to the control (*cf.* Figure 1C). Clonogenicity tests were also carried out on another HPV-positive line, the Mel80 line.

Figures 2A, 2B and 2C illustrate the results obtained with Mel 80 cells.

Cidofovir alone had an effect on Me180 cells when used at a concentration of at least 1 $\mu$g/mL. At the concentration of 1 $\mu$g/mL, Mel 80 cell survival reduced by about 30% (*cf.*

Figure 2A). When the concentration of cidofovir reached 5 $\mu$g/mL, the clonogenicity was reduced by about 40% compared to the control (*cf.* Figure 2A).

Cetuximab alone at 10 $\mu$g/mL resulted in a reduction in Me180 cell survival by about 30% compared to the control (*cf.* Figure 2B).

When the antiviral agent was combined with the EGFR antagonist, the clonogenicity was reduced much more significantly, even at low doses of cidofovir.

Cidofovir at a dose of 1 μg/mL with cetuximab at 10 μg/mL and cidofovir at a dose of 10 μg/mL with cetuximab at 10 μg/mL, resulted in a reduction in cell survival of about 60% compared to the control (*cf.* Figure 2C).

Then, clonogenicity tests were carried out on HPV-negative cells.

Figures 3A, 3B and 3C and Figures 4A, 4B and 4C illustrate the results obtained with the C33A cell line (human HPV-negative cervical carcinoma cells) and with the H480 cell line (human HPV-negative lung carcinoma cells), respectively.

Cidofovir at the concentrations of 1 μg/mL and 5 μg/mL caused a slight reduction in C33A cell survival of about 20% compared to the control (*cf.* Figure 3A).

Cetuximab at the concentration of 10 μg/mL also caused a slight reduction in C33A cell survival of about 15% compared to the control (*cf.* Figure 3B).

When cidofovir at 1 μg/mL was combined with cetuximab at 10 μg/mL, a reduction in C33A cell survival of about 10% was obtained compared to the control (*cf.* Figure 3C). Cidofovir at a dose of 10 μg/mL with cetuximab at 10 μg/mL resulted in a reduction in C33A cell survival of about 65% (*cf.* Figure 3C). Cidofovir alone and cetuximab alone had no significant effect on H480 cell survival (*cf.* Figure 4A and B).

When cidofovir at 5, 10, 15, 20, 30 μg/mL was combined with cetuximab at 50 μg/mL, a reduction in cell survival of about 30%, 40%, 50% 70% and 65%, respectively, was obtained compared to the control (*cf.* Figure 4C).

The combination of the two drugs thus had an effect on the survival of HPV-negative cells, but this effect appeared to be smaller than the effect observed on HPV-positive cells and obtained for higher doses of antiviral agent and anti-EGFR agent.

**Bliss additivity model:**

According to the Bliss model, the effect of the cidofovir-cetuximab combination at the tested concentrations was synergistic (delta had a positive value) for the (HPV-positive) HeLa line (*cf.* Figure 5A). It was calculated that the synergy was:

- 45% for a 1 μg/mL cidofovir + 10 μg/mL cetuximab combination;
- 40% for a 5 μg/mL cidofovir + 10 μg/mL cetuximab combination;
- 35% for a 10 μg/mL cidofovir + 10 μg/mL cetuximab combination.

Regarding the (HPV-positive) Me180 cell line, according to the Bliss model, the combination had a synergistic effect for concentrations of 10 μg/mL of cetuximab with 1 or 5 μg/mL of cidofovir (synergy estimated to be 40% and 60%, respectively (*cf.* Figure 5B).

For the (HPV-negative) C33A cell line, the drugs had an additive effect for combinations containing 1 μg/mL of cidofovir (*cf.* Figure 5C). When cetuximab was combined with 5 μg/mL of cidofovir, a synergistic trend was observed.

Therefore, a synergestic effect is obtained when treating HPV-positive tumor cells with a combination of antiviral agent (cidofovir) and EGFR antagonist (cetuximab).

Furthermore, this synergestic effect can be obtained at a low dose of antiviral agent (cidofovir), more particularly at low doses of antiviral agent (cidofovir) and of EGFR inhibitor (cetuximab); which advantageously enables to limit the risk of adverse side effects that the combined preparation may cause.

<u>Clonogenicity tests with an antiviral agent other than cidofovir and/or with an EGFR</u>

<u>inhibitor other than cetuximab</u>

The *in vitro* results showed a reduction in the clonogenicity of HeLa cells when cetuximab was combined with cidofovir, even at low doses of the drugs. An investigation was therefore carried out to examine the specificity of the two drugs used, and whether the synergic anti-tumoral effect was specific to these two drugs.

The results of the clonogenicity tests carried out using the antiviral agent Foscarvir® (foscarnet sodium) instead of cidofovir and/or the EGFR antagonist Tarceva® (erlotinib) instead of cetuximab, are reported below.

**Clonogenecity tests for the combination of cidofovir and erlotinib (Table1)**

**Table 1:** Synopsis of cell survival for HeLa cells, Me180 cells and C33A cells in the presence of cidofovir, or erlotinib, or a combination of the two drugs

| Decrease in living cells (%) | cidofovir | erlotinib | cidofovir + erlotinib |
|---|---|---|---|
| HeLa (HPV+) | minus 15% at 5 $\mu$g/mL cidofovir | minus 50% at 0.1 $\mu$M/mL erlotinib | minus 80% at 5 $\mu$g/mL cidofovir + 0.1 $\mu$M/mL erlotinib |
| Me180 (HPV+) | minus 40% at 5 $\mu$g/mL cidofovir | minus 80% at 0.5 $\mu$M/mL erlotinib | minus 90% at 5 $\mu$g/mL cidofovir + 0.5 $\mu$M/mL erlotinib |
| C33A (HPV-) | minus 20% at 5 $\mu$g/mL cidofovir | minus 10% at 0.1 $\mu$M/mL erlotinib | minus 30% at 5 $\mu$g/mL cidofovir + 0.1 $\mu$M/mL erlotinib |

On (HPV-positive) HeLa cells:

Cidofovir alone at 5 $\mu$g/mL caused a reduction in the clonogenicity of 15% compared to the control. Erlotinib alone at 0.1 $\mu$M/mL caused a reduction in clonogenicity of 50% compared to the control. The combination of erlotinib with cidofovir caused, compared to the control, a reduction in clonogenicity of 80% even at low doses of cidofovir and erlotinib (5 $\mu$g/mL and 0.1 $\mu$M/mL, respectively).

On (HPV-positive) Me180 cells:

Cidofovir alone at 5 $\mu$g/mL caused a reduction in clonogenicity of 40% compared to the control. Erlotinib alone at 0.5 uM/mL caused a reduction in clonogenicity of 80% compared to the control. The combination of erlotinib with cidofovir caused, compared to the control, a reduction in clonogenicity of 90% at doses of cidofovir and erlotinib of 5 $\mu$g/mL and 0.5 $\mu$M/mL, respectively.

On (HPV-negative) C33A cells:

Cidofovir alone at 5 $\mu$g/mL caused a reduction in clonogenicity of 20% compared to the control. Erlotinib alone at 0.1 $\mu$M/mL caused a reduction in clonogenicity of 10% compared to the control. The combination of cidofovir at 5 $\mu$g/ml and erlotinib at 0.1 $\mu$M/mL only caused a reduction in clonogenicity of 30%.
Thus, it appeared to be a synergistic effect between cidofovir and erlotiriib in HPV-positive cells.

**Clonogenecity tests for the combination of foscarnet and cetuximab (Table 2)**

**Table 2:** Synopsis of cell survival of HeLa cells, Me180 cells and C33A cells in the presence of foscarnet, or cetuximab, or a combination of the two drugs

| Decrease in living cells (%) | Foscarnet | cetuximab | foscarnet + cetuximab |
|---|---|---|---|
| HeLa | minus 20% at 200 $\mu$M/mL foscarnet sodium | minus 20% at 50 $\mu$g/mL cetuximab | minus 70% at 200 $\mu$M/mL foscarnet sodium + 50 $\mu$g/mL cetuximab |
| Me180 | minus 35% at 200 $\mu$M/mL foscarnet sodium | minus 50% at 50 $\mu$g/mL cetuximab | minus 70% at 200 $\mu$M/mL Foscarnet sodium + 50 $\mu$g/mL cetuximab |

(continued)

| Decrease in living cells (%) | Foscarnet | cetuximab | foscarnet + cetuximab |
|---|---|---|---|
| C33A | minus 20% at 200 μM/mL foscarnet sodium | minus 30% at 50 μg/mL cetuximab | minus 30% at 200 μM/mL foscarnet sodium + 50 μg/mL cetuximab |

On (HPV+) HeLa cells:

Foscarnet sodium alone at 200 μM/mL caused a reduction in the clonogenicity of 20% compared to the control. Cetuximab alone at 50 μg/mL caused a reduction in clonogenicity of 20% compared to the control. A combination of foscarnet with cetuximab caused, compared to the control, a reduction in clonogenicity of 70% at doses of foscarnet sodium and cetuximab of 200 μM/mL and 50 μg/mL, respectively.

On (HPV+) Me180 cells:

Foscarnet sodium alone at 200 μM/mL caused a reduction in clonogenicity of 35% compared to the control. Cetuximab alone at 50 μg/mL caused a reduction in clonogenicity of 50% compared to the control. A combination of foscarnet with cetuximab caused, compared to the control, a reduction in clonogenicity of 70% at doses of foscarnet sodium and cetuximab of 200 μM/mL and 50 μg/mL, respectively.

On (HPV-) C33A cells:

Foscarnet sodium alone at 200 μM/mL caused a reduction in clonogenicity of 20% compared to the control. Cetuximab alone at 50 μg/mL caused a reduction in clonogenicity of 30% compared to the control. A combination of foscarnet sodium at 200 μM/mL and cetuximab at 50 μg/mL only caused a 30% reduction in clonogenicity.

**Clonogenecity tests for the combination of foscarnet and erlotinib (Table 3)**

Table 3: synopsis of cell survival of HeLa cells, Me180 cells and C33A cells in the presence of erlotinib, or foscarnet, or a combination of the two drugs

| Decrease in living cells (%) | Foscarnet | erlotinib | foscarnet + erlotinib |
|---|---|---|---|
| HeLa | minus 20% at 200 μM/mL foscarnet sodium | minus 50% at 0.1 μM/mL erlotinib | minus 80% at 200 μM/mL foscarnet sodium + erlotinib 0.1 μM/mL |
| Me180 | minus 35% at 200 μM/mL foscarnet sodium | minus 50% at 0.1 μM/mL erlotinib | minus 80% at 200 μM/mL foscarnet sodium + 0.1 μM/mL erlotinib |
| C33A | minus 20% at 200 μM/mL foscarnet sodium | minus 10% at 0.1 μM/mL erlotinib | minus 20% at 200 μM/mL foscarnet sodium + 0.1 μM/mL erlotinib |

On HeLa (HPV+ cells:

Foscarnet sodium alone at 200 μg/mL caused a reduction in clonogenicity of 20% compared to the control. Erlotinib alone at 0.1 μM/mL caused a reduction in clonogenicity of 50% compared to the control. A combination of foscarnet with erlotinib caused, compared to the control, a reduction in clonogenicity of 80% at doses of foscarnet sodium and erlotinib of 200 μM/mL and 0.1 μM/mL, respectively.

On Me180 (HPV+) cells:

Foscarnet sodium alone at 200 $\mu$M/mL caused a reduction in clonogenicity of 35% compared to the control. Erlotinib alone at 0.1 $\mu$M/mL caused a reduction in clonogenicity of 50% compared to the control. A combination of foscarnet with erlotinib caused, compared to the control, a reduction in clonogenicity of 80% at doses of foscarnet sodium and erlotinib of 200 $\mu$M/mL and 0.1 $\mu$M/mL, respectively.

On C33A HPV-) cells:

Foscarnet sodium alone at 200 $\mu$M/mL caused a reduction in clonogenicity of 20% compared to the control. Erlotinib alone at 0.1 $\mu$M/mL caused a reduction in clonogenicity of 10% compared to the control. A combination of foscarnet sodium at 200 $\mu$M/mL and erlotinib at 0.1 $\mu$M/mL only caused a reduction in clonogenicity of 20%.

DISCUSSION

The effects of a combination of an EGFR inhibitor, cetuximab (Erbitux®), with an antiviral agent, cidofovir (Vistide®), on tumor growth as a function of the HPV status of cells have been examined.
The clonogenicity tests carried out on HPV-positive cell lines demonstrate that Cidofovir with cetuximab exhibit a synergistic action *in vitro.*
The combination has also been tested on other lines: the Me180 (HPV-positive) cell line, the G33A (HPV-negative) and the H460 (HPV-negative) cell line.
Regarding HPV-negative cell lines, the action of the combination was smaller than with the HPV-positive cell lines and was obtained for higher doses.
It thus appears that cidofovir with cetuximab display a synergistic action, which is especially apparent on HPV-positive cells, at least at the lowest doses of antiviral agent and/or EGFR antagonist. The Bliss model was used to reinforce this clonogenic observation. It should be noted that the Bliss method was selected over other methods employed to study a combination of two drugs (the Chou combination index or the isobologram method) as it allows a simple objectivisation of the supra-additive effect of a combination even when one of the treatments is not active alone. In the Bliss model, the cidofovir-cetuximab combination is synergistic for the HeLa (HPV-positive) line; it is partially synergistic and additive for the C33A line.
In order to understand whether this synergic anti-tumor effect obtained for the combination was specific for the two drugs used, clonogenicity tests were carried out on the previously studied lines but using another antiviral agent and/or another EGFR inhibitor (*cf.* tables 1, 2 arid 3 above).
These tests showed that the synergic anti-tumor effect is not specific of the drugs used but rather, it is specific of the mechanism of combining an antiviral agent with an EGFR inhibitor on neoplastic cells, more particularly on HPV-positive neoplastic cells.

## EXAMPLE 2: IN VIVO ASSAYS (mice)

### Material and Methods

Unless otherwise indicated, materials and methods are those described in example 1 above. The *in vivo* experiments were carried out at the Institut Gustave Roussy (94805 Villejuif; France), with the approval of the French Ministry of Agriculture.
Female nude mice came from a January brood (CERT 53940, le Geneset St Isle, France). 10- to 12-week old female nude mice were injected subcutaneously (into the flank) with $2 \times 10^6$ live HeLa cells suspended in 100 $\mu$L of culture medium without Fetal Calf Serum (FCS) to study tumor growth. As soon as the tumor reached 5 mm$^2$, cidofovir was injected intra-peritoneally (100 mg/mouse) for five days (days 1, 2, 3, 4, 5) and cetuximab. (Erbitux®) was also injected intra-peritoneally (1 mg/mouse) for three days (days 1, 4, 7). Tumor volume and weight were monitored twice a week. The mice were sacrificed when the tumor reached ten times the initial volume.

### Results and Discussion:

First injection of drugs in mice causes a small weight loss during the week of treatment, but then the weight is back and constant (*cf.* Figure 6A).
Regarding the evolution of tumor volume, the injection of cetuximab alone or of cifovir alone does not reduce tumor growth compared to the control group, whereas mice injected with cidofovir in combination with cetuximab showed delayed tumor growth compared to the control group (*cf.* Figure 6B).

These *in vivo* data are consistent with the *in vitro* data and suggests synergy between cidofovir and cetuximab.

Similar effects were observed in the ME 180 cell line (*cf*. Figure 7), whereas no synergy could be observed with the HPV-negative C33 cell line *in vivo* (*cf*. Figure 8).

To determine whether the anti-tumor benefice obtained with the combination of cidofovir and cetuximab would be specific of the particular drugs used, similar xenograft experiments have been performed with another antiviral agent (foscarnet sodium; Foscarvir®) and another EGFR inhibitor (erlotinib; Tarceva®). These experiments showed effects comparable to those observed with cidofovir and cetuximab.

Hence, the combination of an antiviral agent and of an EGFR inhibitor seems to have specific interaction mechanisms on neoplastic cells, more particularly on HPV-positive neoplastic cells.

## BIBLIOGRAPHIC REFERENCES

Abdulkarim et al. 2002; Oncogene 21: 2334-2346.

Amine et al. 2006; Radiation Research.166: 600-610.

Broekema and Dikkers 2008; Eur. Arch. Otorhinolaryngol. 265(8): 871-879.

Cunningham et al. 2004; N. Engl. J. Med. 351: 337-345.

Eisenhauer et al. 2009; European Journal of Cancer 45: 228-247.

Kumar et al. 2008; Journal of Clinical Oncology 26(19): 3128-3137 and associated appendix.

Therrasse et al., 2000; J. Natl. Cancer Inst. 92: 205-216..

US 6,217,866 B1

Vermorken et al. 2008; Cancer 112: 2710-2719.

WO 01/32215 A1 in the names of Institut Gustave Roussy and Université Paris-Sud XI.

## Claims

**1.** A combination of biologically active compounds comprising at least one antiviral agent and at least one EGFR inhibitor, for simultaneous, separate or sequential use in the treatment and/or prevention and/or palliation of malignant or pre-malignant neoplasms, wherein said at least one antiviral agent is cidofovir, foscarnet or a salt thereof, wherein said at least one EGFR inhibitor comprises cetuximab, erlotinib or a salt thereof, wherein said neoplasms are Squamous Cell Carcinoma of the Head and Neck (SCCHN), recurrent and/or metastatic SCCHN, cervix tumor(s), vulvar tumor(s), penile tumor(s), anal tumor(s), colorectal tumor(s), lung tumor(s), Non-Small Cell Lung Cancer (NSCLC), locally advanced or metastatic NSCLC, nasopharyngeal tumor(s), and wherein said neoplasms are:

- *Papillomaviridae*-positive and/or EBV-positive, or are
- virus-negative and not virus-associated.

**2.** The combination for use according to claim 1, wherein said at least one antiviral agent is administered on the same day as, but before said at least one EGFR inhibitor.

**3.** The combination for use according to claim 1 or 2, wherein said neoplasms are HPV-positive, and wherein when said at least one antiviral agent comprises cidofovir, cidofovir is administered at a dose leading to a maximum plasmatic concentration of 1-5 μg/mL per administration, and when said at least one antiviral agent comprises foscarnet, foscarnet is administered at a dose leading to a maximum plasmatic concentration of 50-800 nM per administration.

**4.** The combination for use according to any one of claims 1-3, wherein said neoplasms are HPV-positive, and wherein when said at least one EGFR inhibitor comprises cetuximab, cetuximab is administered at a dose leading to a maximum plasmatic concentration of 10-40 μg/mL per administration, and when said at least one EGFR inhibitor comprises erlotinib, erlotinib is administered at a dose leading to a maximum plasmatic concentration of 0.1-2.5 nM per administration.

**5.** The combination for use according to any one of claims 1-4, wherein said neoplasms are:

- *Papillomaviridae*-positive and EBV-negative neoplasms, or *Papillomaviridae*-positive and EBV-positive neoplasms, and/or
- *Papillomaviridae*-negative and EBV-positive neoplasms.

6. The combination for use according to any one of claims 1-5, wherein said *Papillomaviridae* virus is or comprises HR HPV.

7. The combination for use according to any one of claims 1-6, wherein said said *Papillomaviridae* virus is or comprises HPV 16 and/or HPV 18 and/or HPV31 and/or HPV45.

8. The combination for use according to any one of claims 1-7, wherein said said *Papillomaviridae* virus is or comprises HPV 16 and/or HPV 18.

9. The combination for use according to any one of claims 1-4, wherein said neoplasms are virus-negative and are not virus-associated.

10. The combination for use according to any one of claims 1-9, wherein said neoplasms are EGFR-negative neoplasms.

11. The combination for use according to any one of claims 1-9, wherein said neoplasms are EGFR-positive neoplasms.

12. The combination for use according to any one of claims 1-11, for simultaneous, separate or sequential use in the treatment and/or prevention and/or palliation of neoplasms in those subjects, who are non-responsive, or have become resistant to the administration of an EGFR inhibitor and/or to a systemic chemotherapy by cisplatin.

13. The combination for use according to any one of claims 1-11, for simultaneous, separate or sequential use in the treatment and/or prevention and/or palliation of neoplasms in those subjects, for whom the administration of an EGFR inhibitor and/or a systemic chemotherapy by cisplatin is(are) contraindicated.

14. The combination for use according to any one of claims 1-13, which is for sequential use.

**Patentansprüche**

1. Kombination von biologisch aktiven Verbindungen, umfassend mindestens ein antivirales Mittel und mindestens einen EGFR-Inhibitor, zur gleichzeitigen, getrennten oder sequentiellen Verwendung in der Behandlung und/oder Prävention und/oder Linderung von malignen oder pre-malignen Neoplasmen, wobei das mindestens eine antivirale Mittel Cidofovir, Foscarnet oder ein Salz davon ist, wobei dieser mindestens eine EGFR-Inhibitor Cetuximab, Erlotinib oder ein Salz davon umfasst, wobei Neoplasmen Plattenepithelkarzinom vom Kopf und Nacken (Squamous Cell Carcinoma of the Head and Neck, SCCHN), wiederkehrende und/oder metastatische SCCHN, Gebärmutterhalstumor(e), Schamlippentumor(e), penisbezogene(r) Tumor(e), Analtumor(e), Kolorektaltumor(e), Lungentumor(e), nicht-kleinzelliges Lungenkarzinom (Non-Small Cell Lung Cancer, NSCLC), lokal fortgeschrittene oder metastatische NSCLC, Nasen-Rachentumor(e), sind, und wobei die Neoplasmen

   - *Papillomaviridae*-positiv und/oder EBV-positiv sind, oder
   - virus-negativ und nicht-virus-assoziiert sind.

2. Kombination zur Verwendung nach Anspruch 1, wobei das mindestens eine antivirale Mittel am gleichen Tag wie, aber vor dem mindestens einen EGFR-Inhibitor verabreicht wird.

3. Kombination zur Verwendung nach Anspruch 1 oder 2, wobei die Neoplasmen HPV-positiv sind, und wenn das mindestens eine antivirale Mittel Cidofovir umfasst, Cidofovir bei einer Dosis verabreicht wird, welche zu einer maximalen Plasmakonzentration von 1-5 µg/mL pro Verabreichung führt, und wenn das mindestens eine antivirale Mittel Foscarnet umfasst, Foscarnet in einer Dosis verabreicht wird, welche zu einer maximalen Plasmakonzentration von 50-800 nM pro Verabreichung führt.

4. Kombination zur Verwendung nach einem beliebigen der Ansprüche 1-3, wobei die Neoplasmen HPV-positiv sind, und wenn der mindestens eine EGFR-Inhibitor Cetuximab umfasst, Cetuximab in einer Dosis verabreicht wird, welche zu einer maximalen Plasmakonzentration von 10-40 µg/mL pro Verabreichung führt, und wenn der mindestens eine EGFR-Inhibitor Erlotinib umfasst, Erlotinib in einer Dosis verabreicht wird, welche zu einer maximalen Plasmakonzentration von 0,1-2,5 nM pro Verabreichung führt.

5. Kombination zur Verwendung nach einem beliebigen der Ansprüche 1-4, wobei die Neoplasmen

- *Papillomaviridae*-positive und EBV-negative Neoplasmen, oder *Papillomaviridae*-positive und EBV-positive Neoplasmen; und/oder
- *Papillomaviridae*-negative und EBV-positive Neoplasmen sind.

6. Kombination zur Verwendung nach einem beliebigen der Ansprüche 1-5, wobei das *Papillomaviridae*-Virus HR HPV ist oder umfasst.

7. Kombination zur Verwendung nach einem beliebigen der Ansprüche 1-6, wobei das *Papillomaviridae*-Virus HPV16 und/oder HPV 18 und/oder HPV31 und/oder HPV45 ist oder umfasst.

8. Kombination zur Verwendung nach einem beliebigen der Ansprüche 1-7, wobei das *Papillomaviridae*-Virus HPV16 und/oder HPV18 ist oder umfasst.

9. Kombination zur Verwendung nach einem beliebigen der Ansprüche 1-4, wobei die Neoplasmen virus-negativ sind und nicht virus-assoziiert sind.

10. Kombination zur Verwendung nach einem beliebigen der Ansprüche 1-9, wobei die Neoplasmen EGFR-negative Neoplasmen sind.

11. Kombination zur Verwendung nach einem beliebigen der Ansprüche 1-9, wobei die Neoplasmen EGFR-positive Neoplasmen sind.

12. Kombination zur Verwendung nach einem beliebigen der Ansprüche 1-11, zur gleichzeitigen, getrennten oder sequentiellen Verwendung in der Behandlung und/oder Prävention und/oder Linderung von Neoplasmen in den Subjekten, die nicht ansprechen, oder resistent gegenüber der Verabreichung eines EGFR-Inhibitors und/oder gegenüber einer systemischen Chemotherapie durch Cisplatin geworden sind.

13. Kombination zur Verwendung nach einem beliebigen der Ansprüche 1-11, zur gleichzeitigen, getrennten oder sequentiellen Verwendung in der Behandlung und/oder Prävention und/oder Linderung von Neoplasmen in den Subjekten, für welche die Verabreichung eines EGFR-Inhibitors und/oder einer systemischen Chemotherapie durch Cisplatin kontraindiziert ist (sind).

14. Kombination zur Verwendung nach einem beliebigen der Ansprüche 1-13, welche für die sequentielle Verwendung ist.

**Revendications**

1. Combinaison de composés biologiquement actifs comprenant au moins un agent antiviral et au moins un inhibiteur de l'EGFR, destinée à une utilisation simultanée, séparée ou séquentielle dans le traitement et/ou la prévention et/ou la palliation de néoplasmes malins ou pré-malins, dans laquelle ledit au moins un agent antiviral est le cidofovir, le foscarnet ou un sel de ceux-ci,
dans laquelle ledit au moins un inhibiteur de l'EGFR comprend le cetuximab, l'erlotinib ou un sel de ceux-ci,
dans laquelle lesdits néoplasmes sont un carcinome spinocellulaire de la tête et du cou (SCCHN), un SCCHN récurrent et/ou métastatique, une(des) tumeur(s) du col de l'utérus, une(des) tumeur(s) de la vulve, une(des) tumeur(s) du pénis, une(des) tumeur(s) anale(s), une(des) tumeur(s) colorectale(s), une(des) tumeur(s) du poumon, un cancer du poumon non à petites cellules (NSCLC), un NSCLC localement avancé ou métastatique, une(des) tumeur(s) du nasopharynx, et
dans laquelle lesdits néoplasmes sont :

- positifs pour les *Papillomaviridae* et/ou positifs pour l'EBV, ou sont
- négatifs pour les virus et non associés à des virus.

2. Combinaison destinée à une utilisation selon la revendication 1, dans laquelle ledit au moins un agent antiviral est administré le même jour que, mais avant ledit au moins un inhibiteur de l'EGFR.

3. Combinaison destinée à une utilisation selon la revendication 1 ou 2, dans laquelle lesdits néoplasmes sont positifs pour l'HPV, et dans laquelle lorsque ledit au moins un agent antiviral comprend le cidofovir, le cidofovir est administré à une dose menant à une concentration plasmatique maximale de 1 à 5 $\mu$g/ml par administration, et lorsque ledit au moins un agent antiviral comprend le foscarnet, le foscarnet est administré à une dose menant à une concentration plasmatique maximale de 50 à 800 nM par administration.

4. Combinaison destinée à une utilisation selon l'une quelconque des revendications 1 à 3, dans laquelle lesdits néoplasmes sont positifs pour l'HPV, et dans laquelle lorsque ledit au moins un inhibiteur de l'EGFR comprend le cetuximab, le cetuximab est administré à une dose menant à une concentration plasmatique maximale de 10 à 40 $\mu$g/ml par administration, et lorsque ledit au moins un inhibiteur de l'EGFR comprend l'erlotinib, l'erlotinib est administré à une dose menant à une concentration plasmatique maximale de 0,1 à 2,5 nM par administration.

5. Combinaison destinée à une utilisation selon l'une quelconque des revendications 1 à 4, dans laquelle lesdits néoplasmes sont :

   - des néoplasmes positifs pour les *Papillomaviridae* et négatifs pour l'EBV, ou des néoplasmes positifs pour les *Papillomaviridae* et positifs pour l'EBV ; et/ou
   - des néoplasmes négatifs pour les *Papillomaviridae* et positifs pour l'EBV.

6. Combinaison destinée à une utilisation selon l'une quelconque des revendications 1 à 5, dans laquelle ledit virus *Papillomaviridae* est ou comprend un HPV à haut risque (HR HPV) .

7. Combinaison destinée à une utilisation selon l'une quelconque des revendications 1 à 6, dans laquelle ledit virus *Papillomaviridae* est ou comprend l'HPV16 et/ou l'HPV18 et/ou l'HPV31 et/ou l'HPV45.

8. Combinaison destinée à une utilisation selon l'une quelconque des revendications 1 à 7, dans laquelle ledit virus *Papillomaviridae* est ou comprend l'HPV16 et/ou l'HPV18.

9. Combinaison destinée à une utilisation selon l'une quelconque des revendications 1 à 4, dans laquelle lesdits néoplasmes sont négatifs pour les virus et sont non associés à des virus.

10. Combinaison destinée à une utilisation selon l'une quelconque des revendications 1 à 9, dans laquelle lesdits néoplasmes sont des néoplasmes négatifs pour l'EGRF.

11. Combinaison destinée à une utilisation selon l'une quelconque des revendications 1 à 9, dans laquelle lesdits néoplasmes sont des néoplasmes positifs pour l'EGRF.

12. Combinaison destinée à une utilisation selon l'une quelconque des revendications 1 à 11, destinée à une utilisation simultanée, séparée ou séquentielle dans le traitement et/ou la prévention et/ou la palliation de néoplasmes chez des sujets, qui sont non-réactifs, ou sont devenus résistants à l'administration d'un inhibiteur de l'EGFR et/ou à une chimiothérapie systémique par le cisplatine.

13. Combinaison destinée à une utilisation selon l'une quelconque des revendications 1 à 11, destinée à une utilisation simultanée, séparée ou séquentielle dans le traitement et/ou la prévention et/ou la palliation de néoplasmes chez des sujets pour lesquels l'administration d'un inhibiteur de l'EGFR et/ou une chimiothérapie systémique par le cisplatine, est(sont) contre-indiquée(s).

14. Combinaison destinée à une utilisation selon l'une quelconque des revendications 1 à 13, qui est destinée à une utilisation séquentielle.

**Survival of HeLa cells**

Treatment with cidofovir alone:

Treatment with cetuximab alone:

**Figure 1A**

**Figure 1B**

Treatment with cidofovir and cetuximab:

**Figure 1C**

**Survival of Me180 cells**

Treatment with cidofovir alone:

Treatment with cetuximab alone:

**Figure 2A**

**Figure 2B**

Treatment with cidofovir and cetuximab:

**Figure 2C**

**Survival of C33A cells**

Treatment with cidofovir alone:

Treatment with cetuximab alone:

**Figure 3A**

**Figure 3B**

Treatment with cidofovir and cetuximab:

cidofovir 1 µg/mL + cetuximab 10 µg/mL

cidofovir 10 µg/mL + cetuximab 10 µg/mL

**Figure 3C**

**Survival of H460 cells**

Treatment with cidofovir alone:

Treatment with cetuximab alone:

Figure 4A

Figure 4B

Treatment with cidofovir and cetuximab:

Figure 4C

**Effect of the combination of cidofovir with cetuximab**
**(Bliss additivity test)**

On HeLa cells:

Figure 5A

On Me180 cells:

Figure 5B

On C33A cells:

cetuximab 10 µg/mL

Figure 5C

cidofovir 1 µg/mL

cidofovir 5 µg/mL

cidofovir 10 µg/mL

Effect of the combination of cidofovir with cetuximab *in vivo* on HeLa cells:

**Mice weight HeLa**

Legend: control, CDV5, CDV50, CDV100, ERB40, CDV5-ERB40, CDV50-ERB40, CDV100-ERB40 DEC

**Figure 6A**

**HeLa tumor volume**

Legend: ERB, control, CDV 5, CDV 5 - ERB, CDV 50 - ERB 40

ERB 40, CDV 5, control, CDV 50 - ERB 40, CDV 5 - ERB 40

**Figure 6B**

Effect of the combination of cidofovir with cetuximab *in vivo* on Me180 cells:

Me180 tumor volume

control

CDV 50

ERB 40

CDV 50 - ERB 40

—o— control

—o— CDV 50

—o— ERB 40

—o— CDV 50 - ERB 40

**Figure 7**

Effect of the combination of cidofovir with cetuximab *in vivo* on C33A cells:

**Figure 8**

## REFERENCES CITED IN THE DESCRIPTION

**Patent documents cited in the description**

- WO 0132215 A1
- US 5142051 A
- US 6217866 B1
- US 6235883 B

**Non-patent literature cited in the description**

- **VERMORKEN et al.** *Cancer,* 2008, vol. 112, 2710-2719
- *National Cancer Institute Common Terminology Criteria for Adverse Events (CTCAE),* 09 August 2006, http://ctep.cancer.gov/reporting/ctc.html
- **ABDULKARIM et al.** *Oncogene,* 2002, vol. 21, 2334-2346
- **AMINE et al.** *Radiation Research.,* 2006, vol. 166, 600-610
- **BROEKEMA ; DIKKERS.** *Eur. Arch. Otorhinolaryngol,* 2008, vol. 265 (8), 871-879
- **CUNNINGHAM et al.** *N. Engl. J. Med.,* 2004, vol. 351, 337-345
- **EISENHAUER et al.** *European Journal of Cancer,* 2006, vol. 45, 228-247
- **KUMAR et al.** *Journal of Clinical Oncology,* 2008, vol. 26 (19), 3128-3137
- **THERRASSE et al.** *J. Natl. Cancer Inst.,* 2000, vol. 92, 205-216